# EUROPEAN PATENT APPLICATION

(11) **EP 4 484 957 A1**
(43) Date of publication of application: **01.01.2025**
(21) Application number: 23759879.2
(22) Date of filing: 17.02.2023
(51) Int. Cl.: G01N 33/574

(54) **PANCREATIC CANCER DIAGNOSIS ASSISTANCE METHOD, BIOMARKER FOR DETECTING PANCREATIC CANCER, COLORECTAL CANCER DIAGNOSIS ASSISTANCE METHOD, AND BIOMARKER FOR DETECTING COLORECTAL CANCER**

(30) Priority: 24.02.2022 JP 2022026373
(71) Applicant: NIPPON MEDICAL SCHOOL FOUNDATION, Bunkyo-ku Tokyo 113-8602 (JP)
(72) Inventor: HONDA Kazufumi, Tokyo 113-8602 (JP); KASHIRO Ayumi, Tokyo 113-8602 (JP); NAITO Yutaka, Tokyo 113-8602 (JP)
(74) Representative: Germain Maureau
(86) International application number: PCT/JP2023/005662
(87) International publication number: WO 2023/162878

(57) **Abstract**

Provided is a test method capable of accurately determining early pancreatic cancer or colorectal cancer. The method for assisting diagnosis of pancreatic cancer includes detecting and/or quantifying a biomarker in a body fluid sample derived from a subject, and the biomarker is FKBP1B, BID, EREG, STAT5B, UFD1, or the like. The method for assisting diagnosis of colorectal cancer includes detecting and/or quantifying a biomarker in a body fluid sample derived from a subject, and the biomarker is BID, FKBP1B, EREG, MYDGF, TACC3, TBCA, FKBP14, MIF, ARF6, MESD, CIRBP, MANF, ERP29, PDLIM7, DNM1, STAT5B, GRAP2, UFD1, SNCA, PLCB2, SULT1A1, ABHD14B, PRKAR1A, or the like.

## Description

### TECHNICAL FIELD

The present invention relates to a method for assisting diagnosis of pancreatic cancer or a biomarker for detecting pancreatic cancer. The present invention also relates to a method for assisting diagnosis of colorectal cancer or a biomarker for detecting colorectal cancer.

### BACKGROUND ART

Pancreatic cancer is intractable cancer, and the 5-year survival rate is lower than other solid cancers. Since it is difficult to detect early pancreatic cancer and it is often found as advanced cancer, radical surgery can be performed in few cases. The development of biomarkers for efficiently finding early pancreatic cancer that can be radically operated is likely to increase the discovery rate of early pancreatic cancer and improve the pancreatic cancer mortality rate.

Glycan markers such as CA19-9 and DUPAN2 have been put to practical use as biomarkers for pancreatic cancer. However, CA19-9 may not respond to a patient with early pancreatic cancer. In addition, since a Lewis antigen-negative patient does not produce CA19-9, an increase in CA19-9 is not detected even in a patient with advanced pancreatic cancer who is a Lewis antigen-negative patient.

There is also a report on mRNA that is not expressed in normal pancreatic ductal cells but is expressed in invasive pancreatic ductal cancer cells (NON-PATENT DOCUMENT 1). In NON-PATENT DOCUMENT 1, mRNA is extracted from cells obtained by microdissection of a normal pancreatic duct and an invasive pancreatic duct cancer part derived from a surgical specimen, and hybridization is performed on genome wide cDNA microarray to find mRNA. Since these mRNAs have been found based on expression changes in tissues in which cancer has occurred, it is necessary to use tissues collected from subjects in order to use them as diagnostic biomarkers for pancreatic cancer.

On the other hand, it is known that early colorectal cancer can be cured with a high probability, but there is no subjective symptom at an early stage, and when the subjective symptom appears, the disease state has already progressed in many cases. Therefore, screening for the purpose of finding early colorectal cancer patients is performed in general periodic examinations for healthy individuals.

Screening is generally performed by a colorectal cancer test called a fecal occult blood test. In the fecal occult blood test, a trace amount of blood contained in feces due to gastrointestinal bleeding is detected by an immunological method using an anti-human hemoglobin antibody. A subject determined to be positive by the fecal occult blood test is further subjected to a detailed examination such as digital rectal examination, endoscopic examination, or contrast X-ray examination, and an expert such as a doctor comprehensively determines the results to determine colorectal cancer.

Screening is required to have high determination sensitivity, but bleeding in the gastrointestinal tract is mainly observed in colorectal cancer in the intermediate and subsequent stages. Therefore, the probability that an early-stage colorectal cancer patient is determined to be positive by a fecal occult blood test is about 50%.

Colorectal cancer is classified into stages 0 to 4 depending on the existing site of cancer tissue. In colorectal cancer at stages 3 and 4, metastasis to tissues other than the large intestine is observed, whereas in colorectal cancer at stages 0 to 2, cancer tissues exist in the tissues of the large intestine. In colorectal cancer at stage 0, cancer tissues exist in a mucous membrane which is the innermost layer of the large intestine, and in colorectal cancer at stage 1, cancer tissues exist from the mucous membrane to a proper muscle layer. In addition, in colorectal cancer at stage 2, cancer tissues spread from the mucous membrane to the vicinity of the outermost layer of the large intestine beyond the proper muscle layer. It is expected that the type of in vivo metabolites contained in the body fluid sample differs at each stage due to such differences in the existing site of the cancer tissues. However, in the test method described in PATENT DOCUMENT 1, colorectal cancer at all stages is determined based on the measured values of the same type of in vivo metabolites, resulting in a difference in determination accuracy.

### CITATION LIST

### PATENT DOCUMENT

PATENT DOCUMENT 1: Japanese Unexamined Patent Publication No. 2013-246080

### NON-PATENT DOCUMENT

NON-PATENT DOCUMENT 1: Nakamura T, Furukawa Y, Nakagawa H, Tsunoda T, Ohigashi H, Murata K, Ishikawa O, Ohgaki K, Kashimura N, Miyamoto M, Hirano S, Kondo S, Katoh H, Nakamura Y, Katagiri T. Genome-wide cDNA microarray analysis of gene expression profiles in pancreatic cancers using populations of tumor cells and normal ductal epithelial cells selected for purity by laser microdissection. Oncogene. 2004 Mar 25;23(13):2385-400.

### SUMMARY OF THE INVENTION

### TECHNICAL PROBLEM

An object of the present invention is to provide a pancreatic cancer test method capable of accurately determining pancreatic cancer.

Another object of the present invention is to provide a colorectal cancer test method capable of accurately determining colorectal cancer.

### SOLUTION TO THE PROBLEM

A method for assisting diagnosis of colorectal cancer according to the present invention includes detecting and/or quantifying at least one biomarker in a body fluid sample derived from a subject, and the biomarker is a protein selected from the group consisting of FKBP1B, BID, EREG, MYDGF, TACC3, TBCA, FKBP14, MIF, ARF6, MESD, CIRBP, MANF, ERP29, PDLIM7, DNM1, STAT5B, GRAP2, UFD1, SNCA, PLCB2, SULT1A1, ABHD14B, PRKAR1A, PPIB, HAGH, GP6, EIF4EBP1, EGF, CRADD, MPIG6B, LAT, CDC37, TWF2, PPP1R12A, CXCL3, MAX, CDKN2D, EIF4E, IST1, CD69, AIFM1, CRKL, CACYBP, CA13, NT5C3A, DAB2, DBNL, CLEC1B, GOPC, TMED8, GCC1, TBCB, ATPSIF1, NFU1, CORO1A, DTD1, CRYZL1, CXCL5, SRC, BCL2L1, PPP2R5A, DIABLO, and RBPMS2, a fragment thereof, or a post-translational modification form thereof.

A method for assisting diagnosis of colorectal cancer according to the present invention includes detecting and/or quantifying at least one biomarker in a body fluid sample derived from a subject, and the biomarker is a protein selected from the group consisting of BID, TXN, FKBP1B, APEX1, ABHD14B, ATXN10, PRKAR1A, EREG, S100A4, AARSD1, TXNDC5, STX3, CDKN2D, MYDGF, ACYP1, PPIB, TBCA, LRPAP1, PEBP1, SEMA4D, MANF, MSRA, TACC3, HAGH, NSFL1C, LACTB2, FKBP14, BNIP2, ADD1, EIF4EBP1, TALDO1, ANXA3, FABP5, TREML1, SNX9, HSPA1A, SLC9A3R1, CXCL3, TWF2, NUDT16, SOD1, PLPBP, CA2, CD69, EGF, PRDX5, RWDD1, BAX, MIF, CRADD, DXO, DBI, GP6, ANXA4, CA13, OTUD6B, DAG1, PARK7, PSMG4, NT5C3A, IST1, HTRA2, CACYBP, MCFD2, AIFM1, DENR, DPY30, MPIG6B, OGA, MDH1, FKBPL, CIAPIN1, RGS10, UBAC1, GGCT, NUB1, FADD, CRKL, NAA10, CLEC1B, PIK3AP1, CXCL6, TXNRD1, LAT, NUDT2, FHIT, CDC37, HPCAL1, DBNL, DNAJB14, FKBP4, TYMP, GLOD4, SIAE, GPI, FIS1, CRYBB1, RABEP1, THTPA, CEP20, SH3GLB2, NMT1, PDCD5, USP25, QDPR, IL7, PDLIM7, RAB2B, PPP1R12A, MAP2K1, TPMT, DFFA, RAB27B, MESD, GMPR2, TBCB, SERPINB6, CIRBP, TMEM106A, TP53I3, CETN3, COMMD1, NRGN, EIF4E, METAP2, KYAT1, DNPH1, DNAJB6, EIF4B, EDF1, RILP, CHAC2, ARF6, CDC26, ANXA11, ASAH1, NUDT5, RAB33A, AK1, DAB2, ERP29, MAPK9, SERPINB1, GTPBP2, CNPY4, FAM13A, DARS1, PRDX1, NUMB, SYAP1, STK24, JPT2, PPP1R2, NUCB2, ATOX1, DRG2, CHMP1A, CCDC134, MAX, STAT5B, MED18, TMSB10, CASP8, GIPC3, SNX5, PACS2, RABGAP1L, DNAJB2, CASP2, HS1BP3, EIF4G1, IPCEF1, SNAP29, SELP, SCARF1, HPSE, AKT3, NAPRT, ASPSCR1, VASH1, PVALB, PSMD9, DTD1, FAM172A, IMPA1, CXCL8, PPIF, ARL2BP, CALCOCO2, TIMM10, INPP1, CASP3, DNM1, AP3B1, GRAP2, HARS1, TXLNA, MAPKAPK2, TOMM20, PDAP1, SRPK2, HHEX, SH3BP1, MTDH, EVI5, WWP2, KIFBP, GCC1, CHMP6, DTYMK, PCYT2, PDIA4, YWHAQ, PTGES2, CORO1A, PIBF1, PPBP, DCTN6, TSNAX, LYN, GORASP2, LAP3, TMED8, TNFSF14, TBCC, YES1, STIP1, TXNDC9, TPD52L2, PSMD1, and PPP1R14A, a fragment thereof, or a post-translational modification form thereof. A method for assisting diagnosis of pancreatic cancer according to the present invention includes detecting and/or quantifying at least one biomarker in a body fluid sample derived from a subject, the biomarker being a protein selected from the group consisting of FKBP1B, BID, EREG, STAT5B, UFD1, SNCA, PLCB2, AKT2, ARF6, MANF, PPP2R5A, MESD, DOK1, GRAP2, TBCA, TACC3, ERP29, ABHD14B, CIRBP, MYDGF, SULT1A1, CRKL, HAGH, PDLIM7, MIF, FKBP14, DNM1, CD69, NT5C3A, SRC, GOPC, CMIP, CA13, TWF2, DTD1, IRAK4, BCL2L1, MPIG6B, CASP3, MPI, LAT, MTSS2, DAB2, PPIB, TMED8, JPT2, PRKAR1A, AIFM1, CACYBP, and TBCB, a fragment thereof, or a post-translational modification form thereof.

A method for assisting diagnosis of pancreatic cancer according to the present invention includes detecting and/or quantifying at least one biomarker in a body fluid sample derived from a subject, the biomarker being a protein selected from the group consisting of BID, FKBP1B, HAGH, PRKAR1A, ABHD14B, RILP, CD69, ATXN10, APEX1, GOPC, MPIG6B, ARF6, TIMM10, NUDT2, MANF, LAT, TXN, EREG, AARSD1, CASP3, CMIP, PPIB, CDC37, PLPBP, PEBP1, TIMM8A, GP6, EIF4EBP1, JPT2, MTSS2, RGS10, MCFD2, HPCAL1, INPP1, TBCA, DAG1, RWDD1, CDKN2D, NAPRT, MYDGF, ERP29, RAB27B, PCYT2, PRDX5, LACTB2, TALDO1, MSRA, NT5C3A, TACC3, FHIT, CRYGD, CIRBP, TPMT, HSPA1A, DNAJB14, FKBP14, ACYP1, CRYBB1, MESD, MAP2K1, AIFM1, PDCD5, VASH1, SNCA, EGF, SOD1, IMPA1, PARK7, YWHAQ, TYMP, NFKB1, LPP, BNIP2, GET3, MIF, PRDX1, SNX9, ARL13B, HARS1, TWF2, NAA10, BACH1, STX3, FADD, CRADD, PPP1R2, CXCL3, ANXA3, NRGN, PDIA4, FXYD5, AAMDC, CACYBP, PDAP1, ANXA11, LYN, OTUD6B, GLOD4, TMED8, CNPY4, PPP1R14A, VPS37A, CLEC1B, MPI, FAM172A, PLA2G4A, STAT5B, PPME1, SLC9A3R1, MAX, DNAJB6, CASP2, TREML1, CHMP6, CIAPIN1, CASP8, BCL2L1, EIF4B, TRIAP1, DTYMK, DNM1, NUDT5, UFD1, HTRA2, USP25, NSFL1C, DXO, CA13, CRKL, COMMD1, AK1, CHAC2, KIFBP, PLCB2, S100A4, MAPK9, EDF1, PVALB, PDE5A, NUMB, PPP2R5A, IL7, QDPR, TMSB10, MED18, and EBAG9, a fragment thereof, or a post-translational modification form thereof.

A method for assisting diagnosis of pancreatic cancer according to the present invention includes detecting and/or quantifying at least one biomarker in a body fluid sample derived from a subject, and the biomarker is a protein selected from the group consisting of GP2, CUZD1, CTRL, BST2, VAMP5, PNLIPRP1, CPB1, CELA3A, and SULT2A1, a fragment thereof, or a post-translational modification form thereof.

A method for assisting diagnosis of colorectal cancer according to the present invention includes detecting and/or quantifying at least one biomarker in a body fluid sample derived from a subject, and the biomarker is a protein that is SMTN, a fragment thereof, or a post-translational modification form thereof.

A biomarker for detecting pancreatic cancer according to the present invention includes at least one protein selected from the group consisting of FKBP1B, BID, EREG, STAT5B, UFD1, SNCA, PLCB2, AKT2, ARF6, MANF, PPP2R5A, MESD, DOK1, GRAP2, TBCA, TACC3, ERP29, ABHD14B, CIRBP, MYDGF, SULT1A1, CRKL, HAGH, PDLIM7, MIF, FKBP14, DNM1, CD69, NT5C3A, SRC, GOPC, CMIP, CA13, TWF2, DTD1, IRAK4, BCL2L1, MPIG6B, CASP3, MPI, LAT, MTSS2, DAB2, PPIB, TMED8, JPT2, PRKAR1A, AIFM1, CACYBP, and TBCB, a fragment thereof, or a post-translational modification form thereof.

A biomarker for detecting pancreatic cancer according to the present invention includes at least one protein selected from the group consisting of BID, FKBP 1B, HAGH, PRKAR1A, ABHD14B, RILP, CD69, ATXN10, APEX1, GOPC, MPIG6B, ARF6, TIMM10, NUDT2, MANF, LAT, TXN, EREG, AARSD1, CASP3, CMIP, PPIB, CDC37, PLPBP, PEBP1, TIMM8A, GP6, EIF4EBP1, JPT2, MTSS2, RGS10, MCFD2, HPCAL1, INPP1, TBCA, DAG1, RWDD1, CDKN2D, NAPRT, MYDGF, ERP29, RAB27B, PCYT2, PRDX5, LACTB2, TALDO1, MSRA, NT5C3A, TACC3, FHIT, CRYGD, CIRBP, TPMT, HSPA1A, DNAJB14, FKBP14, ACYP1, CRYBB1, MESD, MAP2K1, AIFM1, PDCD5, VASH1, SNCA, EGF, SOD1, IMPA1, PARK7, YWHAQ, TYMP, NFKB1, LPP, BNIP2, GET3, MIF, PRDX1, SNX9, ARL13B, HARS1, TWF2, NAA10, BACH1, STX3, FADD, CRADD, PPP1R2, CXCL3, ANXA3, NRGN, PDIA4, FXYD5, AAMDC, CACYBP, PDAP1, ANXA11, LYN, OTUD6B, GLOD4, TMED8, CNPY4, PPP1R14A, VPS37A, CLEC1B, MPI, FAM172A, PLA2G4A, STAT5B, PPME1, SLC9A3R1, MAX, DNAJB6, CASP2, TREML1, CHMP6, CIAPIN1, CASP8, BCL2L1, EIF4B, TRIAP1, DTYMK, DNM1, NUDT5, UFD1, HTRA2, USP25, NSFL1C, DXO, CA13, CRKL, COMMD1, AK1, CHAC2, KIFBP, PLCB2, S100A4, MAPK9, EDF1, PVALB, PDE5A, NUMB, PPP2R5A, IL7, QDPR, TMSB10, MED 18, and EBAG9, a fragment thereof, or a post-translational modification form thereof.

A biomarker for detecting pancreatic cancer according to the present invention includes at least one protein selected from the group consisting of GP2, CUZD1, CTRL, BST2, VAMP5, PNLIPRP1, CPB1, CELA3A, and SULT2A1, a fragment thereof, or a post-translational modification form thereof.

A biomarker for detecting colorectal cancer according to the present invention includes at least one protein selected from the group consisting of BID, FKBP1B, EREG, MYDGF, TACC3, TBCA, FKBP14, MIF, ARF6, MESD, CIRBP, MANF, ERP29, PDLIM7, DNM1, STAT5B, GRAP2, UFD1, SNCA, PLCB2, SULT1A1, ABHD14B, PRKAR1A, PPIB, HAGH, GP6, EIF4EBP1, EGF, CRADD, MPIG6B, LAT, CDC37, TWF2, PPP1R12A, CXCL3, MAX, CDKN2D, EIF4E, IST1, CD69, AIFM1, CRKL, CACYBP, CA13, NT5C3A, DAB2, DBNL, CLEC1B, GOPC, TMED8, GCC1, TBCB, ATPSIF1, NFU1, CORO1A, DTD1, CRYZL1, CXCL5, SRC, BCL2L1, PPP2R5A, DIABLO, and RBPMS2, a fragment thereof, or a post-translational modification form thereof.

A biomarker for detecting colorectal cancer according to the present invention includes at least one protein selected from the group consisting of BID, TXN, FKBP 1B, APEX1, ABHD14B, ATXN10, PRKAR1A, EREG, S100A4, AARSD1, TXNDC5, STX3, CDKN2D, MYDGF, ACYP1, PPIB, TBCA, LRPAP1, PEBP1, SEMA4D, MANF, MSRA, TACC3, HAGH, NSFL1C, LACTB2, FKBP14, BNIP2, ADD1, EIF4EBP1, TALDO1, ANXA3, FABP5, TREML1, SNX9, HSPA1A, SLC9A3R1, CXCL3, TWF2, NUDT16, SOD1, PLPBP, CA2, CD69, EGF, PRDX5, RWDD1, BAX, MIF, CRADD, DXO, DBI, GP6, ANXA4, CA13, OTUD6B, DAG1, PARK7, PSMG4, NT5C3A, IST1, HTRA2, CACYBP, MCFD2, AIFM1, DENR, DPY30, MPIG6B, OGA, MDH1, FKBPL, CIAPIN1, RGS10, UBAC1, GGCT, NUB1, FADD, CRKL, NAA10, CLEC1B, PIK3AP1, CXCL6, TXNRD1, LAT, NUDT2, FHIT, CDC37, HPCAL1, DBNL, DNAJB14, FKBP4, TYMP, GLOD4, SIAE, GPI, FIS1, CRYBB1, RABEP1, THTPA, CEP20, SH3GLB2, NMT1, PDCD5, USP25, QDPR, IL7, PDLIM7, RAB2B, PPP1R12A, MAP2K1, TPMT, DFFA, RAB27B, MESD, GMPR2, TBCB, SERPINB6, CIRBP, TMEM106A, TP53I3, CETN3, COMMD1, NRGN, EIF4E, METAP2, KYAT1, DNPH1, DNAJB6, EIF4B, EDF1, RILP, CHAC2, ARF6, CDC26, ANXA11, ASAH1, NUDT5, RAB33A, AK1, DAB2, ERP29, MAPK9, SERPINB1, GTPBP2, CNPY4, FAM13A, DARS1, PRDX1, NUMB, SYAP1, STK24, JPT2, PPP1R2, NUCB2, ATOX1, DRG2, CHMP1A, CCDC134, MAX, STAT5B, MED18, TMSB10, CASP8, GIPC3, SNX5, PACS2, RABGAP1L, DNAJB2, CASP2, HS1BP3, EIF4G1, IPCEF1, SNAP29, SELP, SCARF1, HPSE, AKT3, NAPRT, ASPSCR1, VASH1, PVALB, PSMD9, DTD1, FAM172A, IMPA1, CXCL8, PPIF, ARL2BP, CALCOCO2, TIMM10, INPP1, CASP3, DNM1, AP3B1, GRAP2, HARS1, TXLNA, MAPKAPK2, TOMM20, PDAP1, SRPK2, HHEX, SH3BP1, MTDH, EVI5, WWP2, KIFBP, GCC1, CHMP6, DTYMK, PCYT2, PDIA4, YWHAQ, PTGES2, CORO1A, PIBF1, PPBP, DCTN6, TSNAX, LYN, GORASP2, LAP3, TMED8, TNFSF14, TBCC, YES1, STIP1, TXNDC9, TPD52L2, PSMD1, and PPP1R14A, a fragment thereof, or a post-translational modification form thereof.

A biomarker for detecting colorectal cancer according to the present invention includes SMTN or a fragment thereof.

### ADVANTAGES OF THE INVENTION

According to the present invention, pancreatic cancer can be accurately determined. In addition, according to the present invention, colorectal cancer can be accurately determined.

### BRIEF DESCRIPTION OF THE DRAWING

FIG. 1 is a diagram obtained by extracting pancreatic cancer biomarkers having a difference in Log2X between subjects of 2.5 or more and a Student's t-test of P<1.0×10⁻⁹ in a protein expression list obtained by PEA method.
FIG. 2 is a diagram obtained by extracting colorectal cancer biomarkers having a difference in Log2X between subjects of 2.5 or more and a Student's t-test of P<1.0×10⁻⁹ in a protein expression list obtained by PEA method.

### DESCRIPTION OF EMBODIMENTS

Embodiments of the present invention will be described in detail below while referring to the accompanying drawings. The embodiments are only intended to facilitate the understanding of the principles of the present invention and the scope of the present invention is not limited to the following embodiments. Other embodiments where a person skilled in the art can appropriately replace configurations of the embodiments below are included within the scope of the present invention.

In the present invention, there is provided a method for detecting cancer in a target subject, including determining a possibility of suffering from cancer by measuring an amount of a specific biomarker in a body fluid sample derived from the target subject. The method for detecting cancer in a target subject is to assist diagnosis of cancer by detecting and/or quantifying a biomarker in a body fluid sample derived from the target subject.

The cancer to be detected is pancreatic cancer or colorectal cancer.

The method for detecting cancer is a method for detecting cancer in which the amount of a specific marker described later is measured in a body fluid sample derived from a target subject, and the measured value is used as an index.

The measured value can be compared with a reference value, and a case where the measured value in the target subject is recognized to be larger than the reference value can be used as an index of the presence of cancer. For example, when the measured value is recognized to be larger than the reference value, it is determined that the target subject suffers from or may suffer from cancer.

The present invention provides a biomarker for detecting cancer in a target subject, and the biomarker includes determining the possibility of suffering from cancer by measuring the amount of the biomarker in a body fluid sample derived from the target subject.

In the present invention, a biomarker candidate list is prepared by a proximity extension assay (PEA) method, and proteins with a fold change between pancreatic cancer and healthy individuals of Log2X>2.5 and a Student's t-test of P<1.0×10⁻⁹ by microarray analysis are used as pancreatic cancer biomarkers.

That is, for pancreatic cancer, a biomarker to be measured is selected from the group consisting of FKBP1B, BID, EREG, STAT5B, UFD1, SNCA, PLCB2, AKT2, ARF6, MANF, PPP2R5A, MESD, DOK1, GRAP2, TBCA, TACC3, ERP29, ABHD14B, CIRBP, MYDGF, SULT1A1, CRKL, HAGH, PDLIM7, MIF, FKBP14, DNM1, CD69, NT5C3A, SRC, GOPC, CMIP, CA13, TWF2, DTD1, IRAK4, BCL2L1, MPIG6B, CASP3, MPI, LAT, MTSS2, DAB2, PPIB, TMED8, JPT2, PRKAR1A, AIFM1, CACYBP, and TBCB. These pancreatic cancer biomarkers are very useful for distinguishing cancer patients from healthy individuals.

In the present invention, a biomarker candidate list is prepared by the PEA method, and proteins having an AUC value of 0.93 or more in ROC analysis are used as pancreatic cancer biomarkers.

That is, for pancreatic cancer, the biomarker to be measured is selected from the group consisting of BID, FKBP1B, HAGH, PRKAR1A, ABHD14B, RILP, CD69, ATXN10, APEX1, GOPC, MPIG6B, ARF6, TIMM10, NUDT2, MANF, LAT, TXN, EREG, AARSD1, CASP3, CMIP, PPIB, CDC37, PLPBP, PEBP1, TIMM8A, GP6, EIF4EBP1, JPT2, MTSS2, RGS10, MCFD2, HPCAL1, INPP1, TBCA, DAG1, RWDD1, CDKN2D, NAPRT, MYDGF, ERP29, RAB27B, PCYT2, PRDX5, LACTB2, TALDO1, MSRA, NT5C3A, TACC3, FHIT, CRYGD, CIRBP, TPMT, HSPA1A, DNAJB14, FKBP14, ACYP1, CRYBB1, MESD, MAP2K1, AIFM1, PDCD5, VASH1, SNCA, EGF, SOD1, IMPA1, PARK7, YWHAQ, TYMP, NFKB1, LPP, BNIP2, GET3, MIF, PRDX1, SNX9, ARL13B, HARS1, TWF2, NAA10, BACH1, STX3, FADD, CRADD, PPP1R2, CXCL3, ANXA3, NRGN, PDIA4, FXYD5, AAMDC, CACYBP, PDAP1, ANXA11, LYN, OTUD6B, GLOD4, TMED8, CNPY4, PPP1R14A, VPS37A, CLEC1B, MPI, FAM172A, PLA2G4A, STAT5B, PPME1, SLC9A3R1, MAX, DNAJB6, CASP2, TREML1, CHMP6, CIAPIN1, CASP8, BCL2L1, EIF4B, TRIAP1, DTYMK, DNM1, NUDT5, UFD1, HTRA2, USP25, NSFL1C, DXO, CA13, CRKL, COMMD1, AK1, CHAC2, KIFBP, PLCB2, S100A4, MAPK9, EDF1, PVALB, PDE5A, NUMB, PPP2R5A, IL7, QDPR, TMSB10, MED 18, and EBAG9. These pancreatic cancer biomarkers are very useful for distinguishing cancer patients from healthy individuals.

In the present invention, a biomarker candidate list is prepared by the PEA method, and proteins are selected for which, by microarray analysis, a two-fold or more expression difference is observed between pancreatic cancer and healthy individuals, the fold change is Log2X>1.0 between colorectal cancer and pancreatic cancer, Log2X<0.65 between colorectal cancer and healthy individuals, and a statistically significant difference of P<0.01 is observed between pancreatic cancer and colorectal cancer, but a significant difference of P<0.01 cannot be confirmed between colorectal cancer and healthy individuals.

That is, for pancreatic cancer, the biomarker to be measured is selected from the group consisting of GP2, CUZD1, CTRL, BST2, VAMP5, PNLIPRP1, CPB1, CELA3A, and SULT2A1. These pancreatic cancer biomarkers are very useful in that they can perform the distinguishing in a pancreatic cancer-specific manner.

In the present invention, a biomarker candidate list is prepared by the PEA method, and proteins with a fold change between colorectal cancer and healthy individuals of Log2X>2.5 and a Student's t-test of P<1.0×10⁻⁹ by microarray analysis are used as colorectal cancer biomarkers.

That is, for colorectal cancer, a biomarker to be measured is selected from the group consisting of BID, FKBP1B, EREG, MYDGF, TACC3, TBCA, FKBP14, MIF, ARF6, MESD, CIRBP, MANF, ERP29, PDLIM7, DNM1, STAT5B, GRAP2, UFD1, SNCA, PLCB2, SULT1A1, ABHD14B, PRKAR1A, PPIB, HAGH, GP6, EIF4EBP1, EGF, CRADD, MPIG6B, LAT, CDC37, TWF2, PPP1R12A, CXCL3, MAX, CDKN2D, EIF4E, IST1, CD69, AIFM1, CRKL, CACYBP, CA13, NT5C3A, DAB2, DBNL, CLEC1B, GOPC, TMED8, GCC1, TBCB, ATPSIF1, NFU1, CORO1A, DTD1, CRYZL1, CXCL5, SRC, BCL2L1, PPP2R5A, DIABLO, and RBPMS2. These colorectal cancer biomarkers are very useful for distinguishing cancer patients from healthy individuals.

In the present invention, a biomarker candidate list is prepared by the PEA method, and proteins having an AUC value of 0.93 or more in ROC analysis are used as colorectal cancer biomarkers.

That is, in colorectal cancer, the biomarker to be measured is selected from the group consisting of BID, TXN, FKBP1B, APEX1, ABHD14B, ATXN10, PRKAR1A, EREG, S100A4, AARSD1, TXNDC5, STX3, CDKN2D, MYDGF, ACYP1, PPIB, TBCA, LRPAP1, PEBP1, SEMA4D, MANF, MSRA, TACC3, HAGH, NSFL1C, LACTB2, FKBP14, BNIP2, ADD1, EIF4EBP1, TALDO1, ANXA3, FABP5, TREML1, SNX9, HSPA1A, SLC9A3R1, CXCL3, TWF2, NUDT16, SOD1, PLPBP, CA2, CD69, EGF, PRDX5, RWDD1, BAX, MIF, CRADD, DXO, DBI, GP6, ANXA4, CA13, OTUD6B, DAG1, PARK7, PSMG4, NT5C3A, IST1, HTRA2, CACYBP, MCFD2, AIFM1, DENR, DPY30, MPIG6B, OGA, MDH1, FKBPL, CIAPIN1, RGS10, UBAC1, GGCT, NUB1, FADD, CRKL, NAA10, CLEC1B, PIK3AP1, CXCL6, TXNRD1, LAT, NUDT2, FHIT, CDC37, HPCAL1, DBNL, DNAJB14, FKBP4, TYMP, GLOD4, SIAE, GPI, FIS1, CRYBB1, RABEP1, THTPA, CEP20, SH3GLB2, NMT1, PDCD5, USP25, QDPR, IL7, PDLIM7, RAB2B, PPP1R12A, MAP2K1, TPMT, DFFA, RAB27B, MESD, GMPR2, TBCB, SERPINB6, CIRBP, TMEM106A, TP53I3, CETN3, COMMD1, NRGN, EIF4E, METAP2, KYAT1, DNPH1, DNAJB6, EIF4B, EDF1, RILP, CHAC2, ARF6, CDC26, ANXA11, ASAH1, NUDT5, RAB33A, AK1, DAB2, ERP29, MAPK9, SERPINB1, GTPBP2, CNPY4, FAM13A, DARS1, PRDX1, NUMB, SYAP1, STK24, JPT2, PPP1R2, NUCB2, ATOX1, DRG2, CHMP1A, CCDC134, MAX, STAT5B, MED18, TMSB10, CASP8, GIPC3, SNX5, PACS2, RABGAP1L, DNAJB2, CASP2, HS1BP3, EIF4G1, IPCEF1, SNAP29, SELP, SCARF1, HPSE, AKT3, NAPRT, ASPSCR1, VASH1, PVALB, PSMD9, DTD1, FAM172A, IMPA1, CXCL8, PPIF, ARL2BP, CALCOCO2, TIMM10, INPP1, CASP3, DNM1, AP3B1, GRAP2, HARS1, TXLNA, MAPKAPK2, TOMM20, PDAP1, SRPK2, HHEX, SH3BP1, MTDH, EVI5, WWP2, KIFBP, GCC1, CHMP6, DTYMK, PCYT2, PDIA4, YWHAQ, PTGES2, CORO1A, PIBF1, PPBP, DCTN6, TSNAX, LYN, GORASP2, LAP3, TMED8, TNFSF14, TBCC, YES1, STIP1, TXNDC9, TPD52L2, PSMD1, and PPP1R14A. These colorectal cancer biomarkers are very useful for distinguishing cancer patients from healthy individuals.

In the present invention, proteins having high specificity for colorectal cancer in which a four-fold or more expression difference can be confirmed between colorectal cancer and healthy individuals with a statistically significant difference of 1.0×10⁻⁶ or less, but a two-fold or more expression difference is also observed between colorectal cancer and pancreatic cancer are used as colorectal cancer biomarkers.

That is, in colorectal cancer, the biomarker to be measured is SMTN. This colorectal cancer biomarker is very useful in that it can perform the distinguishing in a colorectal cancer-specific manner.

In the present invention, cancer in a target subject is detected, and the target subject is a mammal including human. Examples of the target subject include human, monkey, dog, cat, rat, and mouse, and the target subject is preferably human.

In the present invention, a biomarker in a body fluid sample derived from a subject is detected and/or quantified to assist diagnosis of cancer, and this diagnosis assistance includes measuring the amount of the biomarker and determining the presence or absence of cancer.

In the measuring of the amount of the biomarker, the amount of the biomarker is measured in a body fluid sample derived from a target subject.

The body fluid sample is not particularly limited. Examples thereof include whole blood, serum, plasma, lymph fluid, urine, and saliva, and the body fluid sample is preferably serum or plasma.

A biopsy sample is a sample obtained by, for example, fine needle aspiration (FNA) cytology, endoscopic biopsy, needle biopsy, or urine cytology. The biopsy sample may be a biopsy sample that is known to contain tumor cells or may contain tumor cells. The body fluid sample may be frozen, fixed, and/or permeabilized, and may be, for example, a formalin-fixed paraffin-embedded (FFPE) sample, and/or a freeze-embedded sample. A collection method and a preparation method of the body fluid sample are not particularly limited, and can be performed according to a known method.

The measurement of the amount of the biomarker is not particularly limited, and can be performed according to a known method. Specifically, for example, a mass spectrometry, a method using an antibody capable of specifically recognizing a marker to be measured, a high performance liquid chromatography, an enzyme activity measurement method, and the like are preferred examples.

Examples of the mass spectrometry include a method using a matrix-assisted laser desorption/ionization (MALDI) mass spectrometer, an electrospray ionization (ESI) mass spectrometer, and an electron ionization (EI) mass spectrometer. A preferred mass spectrometry is a MALDI-TOF MS using a MALDI mass spectrometer.

As the enzyme activity measurement method, a single-molecule enzyme activity measurement method is suitable.

Examples of the method using an antibody (immunological measurement method) include Western blotting, radioimmunoassay (RIA), enzyme immunoassay (ELISA, EIA), luminescence immunoassay, fluorescence immunoassay, and PEA method. The antibody used in such a method is an antibody that binds to a biomarker, preferably an antibody that specifically binds to a biomarker. The antibody has a characteristic of being able to detect or capture a biomarker with good sensitivity and specificity. Here, the antibody also includes an antigen-binding fragment thereof. The antibody can be prepared by a method well known to those skilled in the art, and for example, a polyclonal antibody or a monoclonal antibody can be used. Also, the antibody can be used by being labeled or immobilized according to a generally known method.

An antigen-binding fragment of an antibody means a fragment that is a part of an antibody and has specific binding to a target protein like an antibody. Specific examples thereof include Fab, F(ab')2, Fab', a single-chain antibody (scFv), a disulfide stabilized antibody (dsFv), a dimerized V-region fragment (Diabody), and a peptide containing CDR.

The determining of the presence or absence of cancer is determining (or evaluating) the presence or absence of cancer in vitro based on the measured amount of the biomarker. Specifically, the measured value of the amount of the biomarker is compared with a reference value, and a case where the measured value is recognized to be larger than the reference value is used as an index of the presence of cancer. The determination method can be, for example, a method of determining (or evaluating) that cancer is present or there is a possibility that cancer is present in the subject when the measured value of the amount of the biomarker is recognized to be larger than the reference value. Here, the determination refers to evaluating the presence of cancer or the possibility of the presence of cancer in the subject based on the measurement result obtained in the detection method.

The reference value is, for example, a value by which a positive sample and negative samples can be distinguished with high probability based on results obtained by measuring and comparing the measured value of the amount of the biomarker in a normal body fluid sample or the amount of the biomarker contained in a body fluid sample in which the presence of cancer is confirmed (the positive sample) and the amount of the biomarker contained in a plurality of normal body fluid samples (the negative samples).

In some embodiments, the normal body fluid sample is a body fluid sample derived from a healthy individual. The healthy individual refers to an individual who does not suffer from at least cancer, preferably a healthy individual. Furthermore, the healthy individual is required to be the same biological species as the target subject. For example, when the target subject to be subjected to detection is a human, a healthy individual must also be a human. It is preferred that the physical conditions of the healthy individual are the same as or approximate to those of the target subject in which cancer should be detected. The physical conditions are, for example, race, sex, age, height, and weight in the case of a human.

A determination method in a case where the reference value is a measured value of the amount of the biomarker in a normal body fluid sample can be, for example, a method of determining (or evaluating) that cancer is present or there is a possibility that cancer is present in the subject when the measured value of the amount of the biomarker is compared with the reference value and is recognized to be statistically significantly larger than the reference value.

The term being "statistically significantly" includes, for example, a case where the risk rate (significance level) of the obtained value is less than 5%, 1%, 0.1%, 0.01%, 0.001%, or 0.0001%. Therefore, being statistically significantly large for the measured value means that there is a significant difference between the amount of the biomarker obtained from the body fluid sample derived from the target subject and the reference value when the quantitative difference between the both is statistically processed, and the amount of the biomarker in the body fluid sample derived from the target subject is relatively larger than the reference value. For example, the case of being statistically significantly large for the amount of the biomarker corresponds to a case where the amount of the biomarker obtained from the body fluid sample derived from the target subject is, for example, twice or more, preferably three times or more, more preferably five times or more larger than the reference value (for example, the amount of the biomarker of the normal site). A method for assaying statistical processing is not particularly limited as long as a known assay method capable of determining the presence or absence of significance is appropriately used. For example, a student's t-test method, a multiple comparison test method, or the like can be used.

If the measured value of the amount of the biomarker in the body fluid sample derived from the target subject is statistically significantly larger than the reference value, it is evaluated that cancer is present or there is a possibility that cancer is present in the target subj ect.

A determination method in a case where the reference value is a value in which the amount of the biomarker contained in the body fluid sample in which the presence of cancer is confirmed (positive sample) and the amount of the biomarker contained in a plurality of normal body fluid samples (negative samples) are measured and compared, and the positive sample and the negative sample can be distinguished with high probability based on the result can be, for example, a method of determining (or evaluating) that cancer is present or there is a possibility that cancer is present in the subject when the measured value of the amount of the biomarker is recognized to be larger than the reference value.

The reference value can be obtained by statistical processing or the like. The statistical processing is not particularly limited, and examples thereof include an analysis using a receiver-operating-characteristics (ROC) curve. Here, as a method of determining the optimum threshold (cut-off value) as the reference value from the ROC curve, a method using Youden index (sensitivity + specificity - 1) is common. In this method, since a point at which the Youden index (sensitivity + specificity - 1) can be maximized is a point at which both sensitivity and specificity show a balanced value, it is preferred that a value indicating this diagnostic result is set as a cut-off value and adopted as a reference value.

When the measured value of the amount of the biomarker in the body fluid sample of the subject is larger than the reference value, it is evaluated that cancer is present or there is a possibility that cancer is present in the subject.

In addition, in the present invention, a kit for detecting cancer can be provided. The cancer detection kit can be directly or indirectly used, for example, for determining or evaluating the presence or absence of cancer in a subject, or for screening for a candidate substance useful for preventing, improving, or treating cancer.

The cancer detection kit includes a substance capable of specifically recognizing a biomarker or specifically binding the biomarker. Examples of the substance capable of specifically recognizing a biomarker or specifically binding to the biomarker include antibodies. The antibody may be bound to a solid phase carrier. In addition to the antibody, the cancer detection kit may include at least one or more selected from a labeled secondary antibody, a substrate necessary for detection of a label, a carrier, a washing buffer, a sample diluent, an enzyme substrate, a reaction stop solution, a protein as a purified standard substance, an instruction manual, and the like. The antibody capable of specifically recognizing a biomarker or specifically binding to the biomarker is as described above.

When the biomarker has enzyme activity, the cancer detection kit may include at least one or more selected from a sample diluent for measuring the enzyme activity, an enzyme substrate, a reaction stop solution, a protein as a purified standard substance, an instruction manual, and the like.

The cancer detection kit can be used in a method using an antibody in measuring the amount of a biomarker in a body fluid sample (for example, Western blotting, ELISA, radioimmunoassay (RIA), luminescence immunoassay, fluorescence immunoassay, PEA method, or the like).

For example, the amounts of biomarkers present in a biological sample of a healthy individual and a biological sample of a target subject are measured using a cancer detection kit, and when there is a significant difference between the amounts of biomarkers, it is possible to determine and/or diagnose cancer morbidity in a subject.

**[Table 1]**

| Number | Name | Length | Number | Name | Length | Number | Name | Length | Number | Name | Length | Number | Name | Length | Number | Name | Length |
|---|---|---|---|---|---|---|---|---|---|---|---|---|---|---|---|---|---|
| 1 | FKBP1B | 108 | 51 | HILP | 401 | 101 | HARS1 | 509 | 151 | NUMB | 651 | 201 | WWP2 | 870 | 251 | ASAH1 | 411 |
| 2 | BID | 241 | 52 | ATXN10 | 475 | 102 | NAA10 | 235 | 152 | IL7 | 177 | 202 | PTGES2 | 377 | 252 | RAB33A | 237 |
| 3 | EREG | 169 | 53 | APEX1 | 318 | 103 | BACH1 | 736 | 153 | QDPR | 244 | 203 | PIBF1 | 786 | 253 | SERPINB1 | 379 |
| 4 | STAT5B | 787 | 54 | TIMM10 | 90 | 104 | STX3 | 289 | 154 | TMSB10 | 44 | 204 | PPBP | 128 | 254 | GTPBP2 | 602 |
| 5 | UFD1 | 307 | 55 | NUDT2 | 147 | 105 | FADD | 208 | 155 | MFD18 | 208 | 205 | DCTN6 | 190 | 255 | FAM13A | 1023 |
| 6 | SNCA | 140 | 56 | TXN | 105 | 106 | CRADD | 199 | 156 | EBAG9 | 213 | 206 | TSNAX | 290 | 256 | DARS1 | 501 |
| 7 | PLCB2 | 1185 | 57 | AARSD1 | 412 | 107 | PPP1R2 | 206 | 157 | GP2 | 537 | 207 | GORASP2 | 452 | 257 | SYAP1 | 352 |
| 8 | AKT2 | 481 | 58 | CDC37 | 378 | 108 | CXCL3 | 107 | 158 | CUZD1 | 607 | 208 | LAP3 | 519 | 258 | STK24 | 443 |
| 9 | ARF6 | 175 | 59 | PLPBP | 285 | 109 | ANXA3 | 323 | 159 | CTRL | 264 | 209 | TXNDC5 | 432 | 259 | NUCB2 | 421 |
| 10 | MANF | 182 | 60 | PEBP1 | 187 | 110 | NRGN | 78 | 160 | BST2 | 180 | 210 | LRPAP1 | 357 | 260 | ATOX1 | 68 |
| 11 | PPP2R5A | 486 | 61 | TIMM8A | 97 | 111 | PDIA4 | 645 | 161 | VAMP5 | 116 | 211 | SEMA4D | 862 | 261 | DRG2 | 364 |
| 12 | MESD | 234 | 62 | GP6 | 620 | 112 | FXYD5 | 178 | 162 | PNLIPRP1 | 467 | 212 | ADD1 | 799 | 262 | CHMP1A | 240 |
| 13 | DOK1 | 481 | 63 | EIF4EBP1 | 118 | 113 | AAMDC | 168 | 163 | CPB1 | 417 | 213 | FABP5 | 135 | 263 | CCDC134 | 229 |
| 14 | GRAP2 | 330 | 64 | RGS10 | 181 | 114 | PDAP1 | 181 | 164 | CELA3A | 270 | 214 | NUDT16 | 227 | 264 | GIPC3 | 312 |
| 15 | TBCA | 129 | 65 | MCFD2 | 127 | 115 | ANXA11 | 505 | 165 | SULT2A1 | 285 | 215 | CA2 | 260 | 265 | SNX5 | 404 |
| 16 | TACC3 | 838 | 66 | HPCAL1 | 193 | 116 | LYN | 512 | 166 | PPP1R12A | 1030 | 216 | BAX | 221 | 266 | PACS2 | 904 |
| 17 | ERP29 | 261 | 67 | INPP1 | 399 | 117 | OTUD6B | 293 | 167 | EIF4E | 248 | 217 | DBS | 148 | 267 | RABGAP1L | 1051 |
| 18 | ABHD14B | 210 | 68 | DAG1 | 895 | 118 | GI.OD4 | 364 | 168 | isrt | 379 | 218 | ANXA4 | 321 | 268 | DNAJB2 | 324 |
| 19 | CIRBP | 297 | 69 | RWDD1 | 243 | 119 | CNPY4 | 248 | 169 | DBNL | 439 | 219 | PSMG4 | 162 | 269 | TNFSF14 | 240 |
| 20 | MYDGF | 173 | 70 | CDKN2D | 166 | 120 | PPP1R14A | 147 | 170 | GCC1 | 775 | 220 | DENR | 198 | 270 | TBCC | 346 |
| 21 | SULT1A1 | 295 | 71 | NAPRT | 538 | 121 | VPS37A | 397 | 171 | ATP5IF1 | 106 | 221 | DPY30 | 99 | 271 | YES1 | 543 |
| 22 | CRKL | 303 | 72 | RAB27B | 218 | 122 | CLEC1B | 229 | 172 | NFU1 | 254 | 222 | OGA | 916 | 272 | STIP1 | 590 |
| 23 | HAGH | 308 | 73 | PCYT2 | 407 | 123 | FAM172A | 416 | 173 | CORO1A | 461 | 223 | MDH1 | 352 | 273 | TXNDC9 | 226 |
| 24 | PDLIM7 | 457 | 74 | PRDX5 | 162 | 124 | PLA2G4A | 749 | 174 | CRYZL1 | 349 | 224 | FKBPL | 349 | 274 | TPD52L2 | 229 |
| 25 | MIF | 115 | 75 | LACTB2 | 288 | 125 | PPME1 | 400 | 175 | CXCL5 | 114 | 225 | UBAC1 | 405 | 275 | PSMD1 | 953 |
| 26 | FKBP14 | 211 | 76 | TALDO1 | 337 | 126 | SLC9A3R1 | 358 | 176 | DIABLO | 239 | 226 | GGCT | 188 | 276 | SMTN | 1009 |
| 27 | DNM1 | 867 | 77 | MSRA | 162 | 127 | MAX | 160 | 177 | RBPMS2 | 209 | 227 | NUB1 | 615 | | | |
| 28 | CD69 | 199 | 78 | FHIT | 147 | 128 | DNAJB6 | 326 | 178 | HS1BP3 | 392 | 228 | PIK3AP1 | 805 | | | |
| 29 | NT5C3A | 331 | 79 | CRYGD | 174 | 129 | CASP2 | 312 | 179 | EIF4G1 | 1606 | 229 | CXCL6 | 114 | | | |
| 30 | SRC | 536 | 80 | TPMT | 245 | 130 | TREML1 | 311 | 180 | IPCEF1 | 438 | 230 | TXNRD1 | 649 | | | |
| 31 | GOPC | 462 | 81 | HSPA1A | 641 | 131 | CHMP6 | 201 | 181 | SNAP29 | 258 | 231 | FKBP4 | 459 | | | |
| 32 | CMIP | 773 | 82 | DNAJB14 | 379 | 132 | CIAPIN1 | 312 | 182 | SELP | 830 | 232 | SIAE | 523 | | | |
| 33 | CA13 | 262 | 83 | ACYP1 | 99 | 133 | CASP8 | 538 | 183 | SCARF1 | 830 | 233 | GPI | 597 | | | |
| 34 | TWF2 | 349 | 84 | CRYBB1 | 252 | 134 | EIF4B | 616 | 184 | HPSE | 543 | 234 | FIS1 | 152 | | | |
| 35 | DTD1 | 213 | 85 | MAP2K1 | 393 | 135 | TRIAP1 | 76 | 185 | AKT3 | 479 | 235 | RABEP1 | 862 | | | |
| 36 | IRAK4 | 460 | 86 | PDCD5 | 125 | 136 | DTYMK | 251 | 186 | ASPSCR1 | 647 | 236 | THTPA | 230 | | | |
| 37 | BCL2L1 | 233 | 87 | VASH1 | 365 | 137 | NUDT5 | 219 | 187 | PSMD9 | 378 | 237 | CEP20 | 198 | | | |
| 38 | MPIG6B | 241 | 88 | EGF | 1207 | 138 | HTRA2 | 458 | 188 | CXCL8 | 99 | 238 | SH3GLB2 | 415 | | | |
| 39 | CASP3 | 277 | 89 | SOD1 | 154 | 139 | USP25 | 1125 | 189 | PPIF | 201 | 239 | NMT1 | 496 | | | |
| 40 | MPI | 423 | 90 | IMPA1 | 336 | 140 | NSFL1C | 372 | 190 | ARL2BP | 163 | 240 | RAB2B | 216 | | | |
| 41 | LAT | 269 | 91 | PARK7 | 189 | 141 | DXO | 396 | 191 | CALCOCO2 | 470 | 241 | DFFA | 331 | | | |
| 42 | MTSS2 | 747 | 92 | YWHAQ | 245 | 142 | COMMD1 | 190 | 192 | AP3B1 | 1094 | 242 | GMPR2 | 427 | | | |
| 43 | DAB2 | 770 | 93 | TYMP | 487 | 143 | AK1 | 210 | 193 | TXLNA | 546 | 243 | SERPINB6 | 395 | | | |
| 44 | PPIB | 216 | 94 | NFKB1 | 969 | 144 | CHAC2 | 184 | 194 | MAPKAPK2 | 400 | 244 | TMEM106A | 262 | | | |
| 45 | TMFD8 | 325 | 95 | LPP | 612 | 145 | KIFBP | 621 | 195 | TOMM20 | 145 | 245 | TP53I3 | 322 | | | |
| 46 | JPT2 | 190 | 96 | BNIP2 | 326 | 146 | S100A4 | 101 | 196 | SRPK2 | 730 | 246 | CETN3 | 191 | | | |
| 47 | PRKAR1A | 381 | 97 | GET3 | 348 | 147 | MAPK9 | 424 | 197 | HHEX | 270 | 247 | METAP2 | 478 | | | |
| 48 | AIFM1 | 613 | 98 | PRDX1 | 199 | 148 | EDF1 | 148 | 198 | SH3BP1 | 701 | 248 | KYAT1 | 516 | | | |
| 49 | CACYBP | 228 | 99 | SNX9 | 595 | 149 | PVALB | 110 | 199 | MTDH | 612 | 249 | DNPH1 | 174 | | | |
| 50 | TBCB | 244 | 100 | ARL13B | 428 | 150 | PDE5A | 875 | 200 | EVI5 | 826 | 250 | CDC26 | 85 | | | |

### Examples

### (1) Example 1

In order to discriminate pancreatic cancer/colorectal cancer from healthy individuals with matched background information such as sex and age, 120 cases of plasma specimens (Table 2) were prepared, and a biomarker candidate list thereof was prepared using a proximity extension assay (PEA) method.

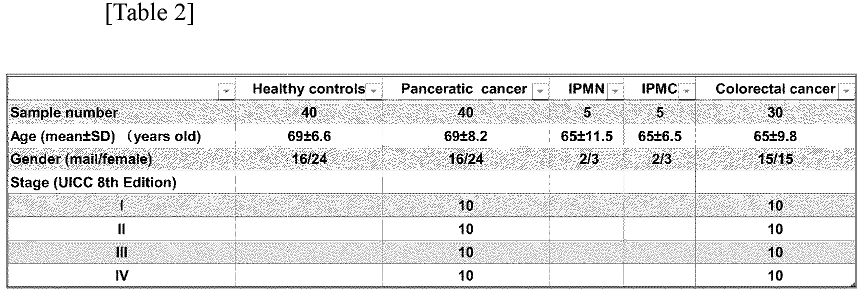

The PEA method is a method in which a sandwich assay is performed with two different antibodies for one antigen, and DNA complementary to two pairs of antibodies for sandwiching is attached to the Fc portion of the antibody. The two pairs of antibodies react with and approach the antigen to form a specific DNA duplex. By amplifying double-stranded DNA and performing quantitative PCR or next generation sequence analysis, the antigen concentration contained in blood or the like can be estimated from the amount of amplified DNA to which a specific barcode is attached. In the present invention, the amounts of 3072 types of antigens contained in the subject blood were simultaneously analyzed using the same method.

### (1) Biomarker candidates for discriminating pancreatic cancer from healthy individual No. 1

"Proteins with a fold change between pancreatic cancer and healthy individuals of Log2X>2.5 and a Student's t-test of P<1.0×10⁻⁹" (Table 3) or "AUC value of 0.93 or more in ROC analysis" (Table 4) were used as pancreatic cancer biomarkers.

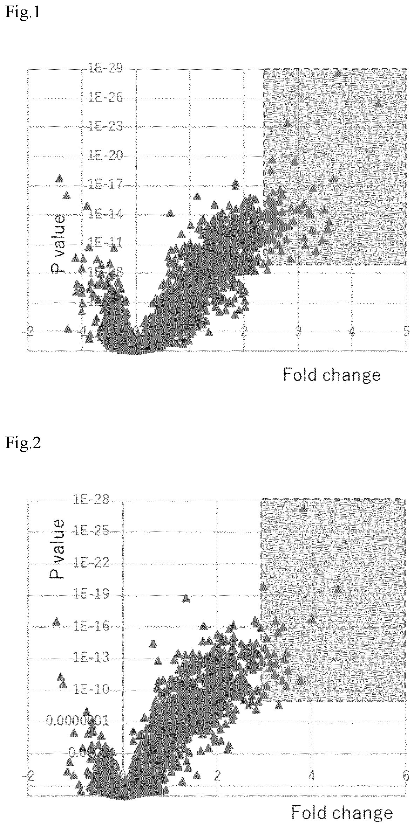

That is, antigens having signal responses worth analyzing were extracted from a list of 3072 antigens in pancreatic cancer patient plasma and healthy individual plasma specimens, which were selected without bias such as age and sex from the protein expression list obtained by the PEA method, and furthermore, a difference in Log2X between the specimens was 2.5 or more and a Student's t-test of P<1.0x 10⁻⁹ were extracted (FIG. 1).

In addition, receiver operating characteristic curve (ROC) analysis was performed, and proteins having an area under curve (AUC) value of 0.93 or more were selected from the protein expression list obtained by the PEA method.

In this way, proteins having either characteristic were defined as pancreatic cancer biomarkers.

### [Table 3]

**[Table 3]**

| Pancreatic cancer biomarker | Expression level (Log2X) | | Expression difference between pancreatic cancer and healthy person (Log2X) | P value |
|---|---|---|---|---|
| | Pancreatic cancer | Healthy | Pancreatic cancer - Healthy | |
| FKBP1B | 5.23 | 0.74 | 4.49 | 3.39E-26 |
| BID | 2.47 | -1.26 | 3.73 | 2.20E-29 |
| EREG | 4.25 | 0.61 | 3.65 | 1.72E-18 |
| STAT5B | 6.03 | 2.45 | 3.58 | 5.40E-14 |
| UFD1 | 6.7 | 3.17 | 3.54 | 2.66E-13 |
| SNCA | 7.47 | 3.99 | 3.48 | 2.66E-15 |
| PLCB2 | 6.01 | 2.57 | 3.44 | 4.04E-12 |
| AKT2 | 6.74 | 3.41 | 3.34 | 4.75E-11 |
| ARF6 | 5.66 | 2.39 | 3.27 | 1.83E-17 |
| MANF | 6.52 | 3.26 | 3.26 | 3.37E-13 |
| PPP2R5A | 4.72 | 1.5 | 3.22 | 7.08E-15 |
| MESO | 5.65 | 2.46 | 3.19 | 3.30E-15 |
| DOK1 | 5.87 | 2.73 | 3.14 | 1.15E-11 |
| GRAP2 | 5.84 | 2.73 | 3.11 | 1.84E-12 |
| TBCA | 4.89 | 1.78 | 3.1 | s.asε^{_}-16 |
| TACC3 | 501 | 2 | 3.01 | 2.03E-15 |
| ERP29 | 5.4 | 2.42 | 2.97 | 2.71 E-15 |
| ABHD14B | 3.42 | 0.48 | 2.93 | 3.01E-20 |
| CIRBP | 5.45 | 2.54 | 2.91 | 3.58E-14 |
| MYDGF | 4.42 | 1.54 | 2.88 | 1.73E-15 |
| SULT1A1 | 5.36 | 2.49 | 2.87 | 2.86E-10 |
| CRKL | 5.57 | 2.73 | 2.84 | 1.34E-13 |
| HAGH | 2.66 | -0.13 | 2.79 | 3.28E-24 |
| PDLIM7 | 5.28 | 2.5 | 2.79 | 2.89E-12 |
| MIF | 4.49 | 1.72 | 2.77 | 1.15E-13 |
| FKBP14 | 3.95 | 1.24 | 2.71 | 4.19E-15 |
| DNM1 | 3.87 | 1.17 | 2.7 | 8.30E-14 |
| CD69 | 4.02 | 1.32 | 2.7 | 6.81E-17 |
| NT5C3A | 5.36 | 2.67 | 2.69 | 1.83E-13 |
| SRC | 5.82 | 3.13 | 2.69 | 4.87E-11 |
| GOPC | 3.95 | 1.29 | 2.66 | 2.36E-17 |
| CMIP | 3.59 | 0.93 | 2.66 | 3.76E-16 |
| CA13 | 5.18 | 2.54 | 2.65 | 6.13E-13 |
| TWF2 | 4.18 | 1.54 | 2.64 | 2.96E-14 |
| DTD1 | 5.77 | 3.13 | 2.64 | 9.01E-12 |
| IRAK4 | 4.88 | 2.26 | 2.63 | 1.20E-11 |
| BCL2L1 | 4.6 | 2.03 | 2.57 | 1.43E-13 |
| MPIG6B | 4.22 | 1.65 | 2.56 | 2.21E-15 |
| CASP3 | 4.59 | 2.03 | 2.56 | 1.65E-14 |
| MPI | 4.12 | 1.57 | 2.55 | 5.01E-14 |
| LAT | 4.68 | 2.13 | 2.55 | 3.73E-15 |
| MTSS2 | 357 | 1.03 | 2.54 | 4.49E-17 |
| DAB2 | 4.97 | 2.44 | 2.53 | 2.65E-12 |
| PPIB | 4.9 | 2.37 | 2.53 | 4.06E-16 |
| TMED8 | 4.23 | 1.7 | 2.53 | 2.17E-15 |
| JPT2 | 3.92 | 1.4 | 2.53 | 6.37E-16 |
| PRKAR1A | 2.84 | 0.32 | 2.52 | 2.03E-20 |
| AIFM1 | 3.6 | 1.09 | 2.51 | 8.00E-15 |
| CACYBP | 421 | 1.7 | 2.5 | 2.23E-13 |
| TBCB | 5.58 | 3.08 | 2.5 | 3.78E-11 |

**[Table 4-1]**

| Pancreatic cancer biomarker | AUC (Pancreatic cancer vs Healthy individual) | Pancreatic cancer biomarker | AUC (Pancreatic cancer vs Healthy individual) |
|---|---|---|---|
| BID | 0.999375 | GET3 | 0.948125 |
| FKBP1B | 0.9975 | MIF | 0.9475 |
| HAGH | 0.99375 | PRDX1 | 0.9475 |
| PRKAR1A | 0.99 | SNX9 | 0.94625 |
| ABHD14B | 0.988125 | ARL13B | 0.94625 |
| RILP | 0.983125 | HARS1 | 0.945625 |
| CD69 | 0.983125 | TWF2 | 0.945625 |
| ATXN10 | 0.983125 | NAA10 | 0.945625 |
| APEX1 | 0.9825 | BACH1 | 0.945625 |
| GOPC | 0.976875 | STX3 | 0.945 |
| MPIG6B | 0.975625 | FADD | 0.944375 |
| ARF6 | 0.975 | CRADD | 0.944375 |
| TIMM10 | 0.974375 | PPP1R2 | 0.944375 |
| NUDT2 | 0.9725 | CXCL3 | 0.94375 |
| MANF | 0.970625 | ANXA3 | 0.94375 |
| LAT | 0.970625 | NRGN | 0.94375 |
| TXN | 0.97 | PDIA4 | 0.943125 |
| EREG | 0.97 | FXYD5 | 0.943125 |
| AARSD1 | 0.969375 | AAMDC | 0.943125 |
| CASP3 | 0.9675 | CACYBP | 0.9425 |
| CMIP | 0.965625 | PDAP1 | 0.9425 |
| PPIB | 0.965 | ANXA11 | 0.9425 |
| CDC37 | 0.9646875 | LYN | 0.9425 |
| PLPBP | 0.964375 | OTUD6B | 0.941875 |
| PEBP1 | 0.963125 | GLOD4 | 0.941875 |
| TIMM8A | 0.963125 | TMED8 | 0.941875 |
| GP6 | 0.9625 | CNPY4 | 0.94125 |
| EIF4EBP1 | 0.9625 | PPP1R14A | 0.94125 |
| JPT2 | 0.9625 | VPS37A | 0.94125 |
| MTSS2 | 0.9625 | CLEC1B | 0.940625 |
| RGS10 | 0.961875 | MPI | 0.940625 |
| MCFD2 | 0.961875 | FAM172A | 0.94 |
| HPCAL1 | 0.96125 | PLA2G4A | 0.94 |
| INPP1 | 0.96125 | STAT5B | 0.94 |

**[Table 4-2]**

| Pancreatic cancer biomarker | AUC (Pancreatic cancer vs Healthy individual) | Pancreatic cancer biomarker | AUC (Pancreatic cancer vs Healthy individual) |
|---|---|---|---|
| TBCA | 0.960625 | PPME1 | 0.939375 |
| DAG1 | 0.960625 | SLC9A3R1 | 0.9390625 |
| RWDD1 | 0.96 | MAX | 0.93875 |
| CDKN2D | 0.959375 | DNAJB6 | 0.93875 |
| NAPRT | 0.959375 | CAS P2 | 0.93875 |
| MYDGF | 0.95875 | TREML1 | 0.938125 |
| ERP29 | 0.958125 | CHMP6 | 0.938125 |
| RAB27B | 0.9575 | CIAPIN1 | 0.938125 |
| PCYT2 | 0.9575 | CASP8 | 0.938125 |
| PRDX5 | 0.9575 | BCL2L1 | 0.9375 |
| LACTB2 | 0.956875 | EIF4B | 0.9375 |
| TALDO1 | 0.95625 | TRIAP1 | 0.9375 |
| MSRA | 0.955625 | DTYMK | 0.936875 |
| NT5C3A | 0.955625 | DNM1 | 0.93625 |
| TACC3 | 0.955 | NUDT5 | 0.93625 |
| FHIT | 0.955 | UFD1 | 0.93625 |
| CRYGD | 0.955 | HTRA2 | 0.93625 |
| CIRBP | 0.954375 | USP25 | 0.93625 |
| TPMT | 0.954375 | NSFL1C | 0.935625 |
| HSPA1A | 0.953125 | DXO | 0.935 |
| DNAJB14 | 0.953125 | CA13 | 0.935 |
| FKBP14 | 0.9525 | CRKL | 0.935 |
| ACYP1 | 0.9525 | COMMD1 | 0.935 |
| CRYBB1 | 0.951875 | AK1 | 0.934375 |
| MESD | 0.951875 | CHAC2 | 0.934375 |
| MAP2K1 | 0.951875 | KIFBP | 0.93375 |
| AIFM1 | 0.95125 | PLCB2 | 0.93375 |
| PDCD5 | 0.95125 | S100A4 | 0.93375 |
| VASH1 | 0.95125 | MAPK9 | 0.93375 |
| SNCA | 0.95125 | EDF1 | 0.933125 |
| EGF | 0.950625 | PVALB | 0.933125 |
| SOD1 | 0.950625 | PDE5A | 0.9325 |
| IMPA1 | 0.950625 | NUMB | 0.93125 |
| PARK7 | 0.949375 | PPP2R5A | 0.93125 |
| YWHAQ | 0.949375 | IL7 | 0.930625 |
| TYMP | 0.949375 | QDPR | 0.930625 |
| NFKB1 | 0.949375 | TMSB10 | 0.930625 |
| LPP | 0.94875 | MED18 | 0.9303125 |
| BNIP2 | 0.948125 | EBAG9 | 0.93 |

### (2) Biomarker candidates for discriminating colorectal cancer from healthy individual No. 1

"Proteins with a fold change between colorectal cancer and healthy individuals of Log2X>2.5 and a Student's t-test of P<1.0×10⁻⁹" (Table 5) or "AUC value of 0.93 or more in ROC analysis" (Table 6) were used as colorectal cancer biomarkers.

That is, antigens having signal responses worth analyzing were extracted from a list of 3072 antigens in colorectal cancer patient plasma and healthy individual plasma specimens, which were selected without bias such as age and sex from the protein expression list obtained by the PEA method, and furthermore, a difference in Log2X between the specimens was 2.5 or more and a Student's t-test of P<1.0×10⁻⁹ were extracted (FIG. 2).

Receiver operating characteristic curve (ROC) analysis was performed, and proteins having an area under curve (AUC) value of 0.93 or more were selected from the protein expression list obtained by the PEA method.

In this way, proteins having either characteristic were defined as colorectal cancer biomarkers.

**[Table 5-1]**

| Colorectal cancer biomarker | Expression level (Log2X) | | Expression difference betweer colorectal cancer and healthy individual (Log2X) | P value |
|---|---|---|---|---|
| BID | 2.56 | -1.26 | 3.83 | 5.35 E-28 |
| FKBP1B | 5.31 | 0.74 | 4.57 | 2.51E-20 |
| EREG | 4.62 | 0.61 | 4.01 | 1.44E-17 |
| MYDGF | 4.79 | 1.54 | 3.25 | 2.47 E-17 |
| TACC3 | 5.41 | 2 | 3.41 | 8.27E-17 |
| TBCA | 5.08 | 1.78 | 3.3 | 3.09E-16 |
| FKBP14 | 4.28 | 1.24 | 3.05 | 1.15 E-15 |
| MIF | 4.74 | 1.72 | 3.03 | 8.67E-15 |
| ARF6 | 5.6 | 2.39 | 3.21 | 2.86E-14 |
| MESD | 5.93 | 2.46 | 3.47 | 3.22E-14 |
| CIRBP | 5.62 | 2.54 | 3.08 | 3.64E-14 |
| MANF | 6.69 | 3.26 | 3.43 | 8.89E-14 |
| ERP29 | 5.57 | 2.42 | 3.14 | 1.98E-13 |
| PDLIM7 | 5.74 | 2.5 | 3.24 | 2.84E-13 |
| DNM1 | 4.31 | 1.17 | 3.15 | 6.67E-13 |
| STAT5B | 5.94 | 2.45 | 3.49 | 1.27E-12 |
| GRAP2 | 5.95 | 2.73 | 3.22 | 3.02E-12 |
| UFD1 | 6.94 | 3.17 | 3.77 | 1.07E-11 |
| SNCA | 7.38 | 3.99 | 3.39 | 1.09E-11 |
| PLCB2 | 6.03 | 2.57 | 3.46 | 3.00E-11 |
| SULT1A1 | 5.54 | 2.49 | 3.05 | 3.73 E-11 |
| ABHD14B | 3.47 | 0.48 | 2.99 | 1.30E-20 |
| PRKAR1A | 3.14 | 0.32 | 2.81 | 2.32E-17 |
| PPIB | 5.22 | 2.37 | 2.85 | 3.85E-17 |
| HAGH | 2.79 | -0.13 | 2.92 | 1.16E-16 |
| GP6 | 4.42 | 1.7 | 2.72 | 3.24E-15 |
| EIF4EBP1 | 4.21 | 1.69 | 2.52 | 3.31E-15 |
| EGF | 3.55 | 0.73 | 2.83 | 3.51E-15 |
| CRADD | 4.77 | 2.2 | 2.57 | 8.98E-15 |
| MPIG6B | 4.41 | 1.65 | 2.76 | 1.05E-14 |

**[Table 5-2]**

| Colorectal cancer biomarker | Expression level (Log2X) | | Expression difference between colorectal cancer and healthy individual | P value |
|---|---|---|---|---|
| LAT | 4.76 | 2.13 | 2.63 | 1.06E-14 |
| CDC37 | 3.97 | 1.33 | 2.64 | 1.22E-14 |
| TWF2 | 4.37 | 1.54 | 2.83 | 2.61E-14 |
| PPP1R12A | 4.57 | 1.85 | 2.72 | 2.84E-14 |
| CXCL3 | 4.43 | 1.89 | 2.54 | 3.32E-14 |
| MAX | 4.33 | 1.43 | 2.9 | 3.80E-14 |
| CDKN2D | 4.66 | 2.03 | 2.62 | 5.26E-14 |
| EIF4E | 3.89 | 1.24 | 2.65 | 6.21E-14 |
| IST1 | 4.57 | 1.9 | 2.67 | 7.61E-14 |
| CD69 | 4.09 | 1.32 | 2.77 | 1.22E-13 |
| AIFM1 | 4.04 | 1.09 | 2.95 | 1.64E-13 |
| CRKL | 5.67 | 2.73 | 2.94 | 1.69E-13 |
| CACYBP | 4.38 | 1.7 | 2.67 | 3.02E-13 |
| CA13 | 5.33 | 2.54 | 2.79 | 3.10E-13 |
| NT5C3A | 5.4 | 2.67 | 2.73 | 3.33E-13 |
| DAB2 | 5.27 | 2.44 | 2.83 | 5.34E-13 |
| DBNL | 4.87 | 2.2 | 2.67 | 8.97E-13 |
| CLEC1B | 3.59 | 0.83 | 2.76 | 9.17E-13 |
| GOPC | 3.84 | 1.29 | 2.56 | 1.59E-12 |
| TMED8 | 4.24 | 1.7 | 2.54 | 4.89E-12 |
| GCC1 | 4.69 | 2.1 | 2.59 | 1.93E-11 |
| TBCB | 5.7 | 3.08 | 2.62 | 2.19E-11 |
| ATP5IF1 | 4.72 | 2.19 | 2.52 | 3.28E-11 |
| NFU1 | 4.84 | 2.2 | 2.64 | 4.92E-11 |
| CORO1A | 3.55 | 0.85 | 2.7 | 5.26E-11 |
| DTD1 | 5.98 | 3.13 | 2.85 | 6.68E-11 |
| CRYZL1 | 5.41 | 2.9 | 2.52 | 7.86E-11 |
| CXCL5 | 4.35 | 1.79 | 2.56 | 9.56E-11 |
| SRC | 6.01 | 3.13 | 2.89 | 1.13E-10 |
| BCL2L1 | 4.68 | 2.03 | 2.65 | 1.15E-10 |
| PPP2R5A | 4.49 | 1.5 | 2.99 | 2.66E-10 |
| DIABLO | 5.04 | 2.31 | 2.74 | 3.10E-10 |
| RBPMS2 | 5.55516 | 2.9779975 | 2.58 | 7.93E-10 |

**[Table 6-1]**

| Colorectal cancer biomarker | AUC (Colorectal cancer vs Healthy individual) | Colorectal cancer biomarker | AUC (Colorectal cancer vs Healthy individual) | Colorectal cancer biomarker | AUC (Colorectal cancer vs Healthy individual) |
|---|---|---|---|---|---|
| BID | 1 | NAA10 | 0.9525 | CHMP1A | 0.940833 |
| TXN | 0.998333 | CLEC1B | 0.9525 | CCDC134 | 0.940833 |
| FKBP1B | 0.988333 | PIK3AP1 | 0.9525 | MAX | 0.940833 |
| APEX1 | 0.985833 | CXCL6 | 0.9525 | STAT5B | 0.940833 |
| ABHD14B | 0.985 | TXNRD1 | 0.9525 | MED18 | 0.940833 |
| ATXN10 | 0.984167 | LAT | 0.951667 | TMSB10 | 0.94 |
| PRKAR1A | 0.98 | NUDT2 | 0.951667 | CASPβ | 0.94 |
| EREG | 0.976667 | FHIT | 0.951667 | GIPC3 | 0.939583 |
| S100A4 | 0.975833 | CDC37 | 0.950833 | SNX5 | 0.939167 |
| AARSD1 | 0.975 | HPCAL1 | 0.950833 | PACS2 | 0.939167 |
| TXNDC5 | 0.975 | DBNL | 0.950833 | RABGAP1L | 0.939167 |
| STX3 | 0.973333 | DNAJB14 | 0.950833 | DNAJB2 | 0.939167 |
| CDKN2D | 0.9725 | FKBP4 | 0.950833 | CASP2 | 0.939167 |
| MYDGF | 0.9725 | TYMP | 0.950833 | HS1BP3 | 0.939167 |
| ACYP1 | 0.970833 | GLOD4 | 0.950833 | EIF4G1 | 0.938333 |
| PPIB | 0.970833 | SIAE | 0.950833 | IPCEF1 | 0.938333 |
| TBCA | 0.97 | GPI | 0.95 | SNAP29 | 0.938333 |
| LRPAP1 | 0.969583 | FIS1 | 0.95 | SELP | 0.938333 |
| PEBP1 | 0.969167 | CRYBB1 | 0.95 | SCARF1 | 0.938333 |
| SEMA4D | 0.968333 | RABEP1 | 0.95 | HPSE | 0.938333 |
| MANF | 0.9675 | THTPA | 0.95 | AKT3 | 0.9375 |
| MSRA | 0.9675 | CEP20 | 0.949167 | NAPRT | 0.9375 |
| TACC3 | 0.965833 | SH3GLB2 | 0.949167 | ASPSCR1 | 0.9375 |
| HAGH | 0.965833 | NMT1 | 0.949167 | VASH1 | 0.9375 |
| NSFL1C | 0.965 | PDCD5 | 0.949167 | PVALB | 0.9375 |
| LACTB2 | 0.965 | USP25 | 0.949167 | PSMD9 | 0.9375 |
| FKBP14 | 0.965 | QDPR | 0.949167 | DTD1 | 0.9375 |
| BNIP2 | 0.964167 | IL7 | 0.949167 | FAM172A | 0.9375 |
| ADD1 | 0.964167 | PDLIM7 | 0.949167 | IMPAL | 0.9375 |
| EIF4EBP1 | 0.964167 | RAB2B | 0.949167 | CXCL8 | 0.9375 |
| TALDO1 | 0.963333 | PPP1R12A | 0.949167 | PPIF | 0.936667 |
| ANXA3 | 0.963333 | MAP2K1 | 0.948333 | ARL2BP | 0.936667 |
| FABP5 | 0.963333 | TPMT | 0.948333 | CALCOCO2 | 0.936667 |
| TREML1 | 0.963333 | DFFA | 0.948333 | TIMM10 | 0.936667 |
| SNX9 | 0.963333 | RAB27B | 0.948333 | INPP1 | 0.936667 |
| HSPA1A | 0.963333 | MESD | 0.948333 | CASP3 | 0.936667 |

**[Table 6-2]**

| Colorectal cancer biomarker | AUC (Colorectal cancer vs Healthy individual) | Colorectal cancer biomarker | AUC (Colorectal cancer vs Healthy individual) | Colorectal cancer biomarker | AUC (Colorectal cancer vs Healthy individual) |
|---|---|---|---|---|---|
| SLC9A3R1 | 0.9625 | GMPR2 | 0.948333 | DNM1 | 0.936667 |
| CXCL3 | 0.9625 | TBCB | 0.9475 | AP3B1 | 0.936667 |
| TWF2 | 0.9625 | SERPINB6 | 0.9475 | GRAP2 | 0.936667 |
| NUDT16 | 0.9625 | CIRBP | 0.9475 | HARS1 | 0.936667 |
| SOD1 | 0.961667 | TMEM106A | 0.9475 | TXLNA | 0.935833 |
| PLPBP | 0.961667 | TP5313 | 0.9475 | MAPKAPK2 | 0.935833 |
| CA2 | 0.960833 | CETN3 | 0.9475 | TOMM20 | 0.935833 |
| CD69 | 0.960833 | COMMD1 | 0.9475 | PDAP1 | 0.935833 |
| EGF | 0.960833 | NRGN | 0.946667 | SRPK2 | 0.935833 |
| PRDX5 | 0.960833 | EIF4E | 0.946667 | HHEX | 0.935 |
| RWDD1 | 0.960833 | METAP2 | 0.946667 | SH3BP1 | 0.935 |
| BAX | 0.960833 | KYAT1 | 0.946667 | MTDH | 0.935 |
| MIF | 0.960833 | DNPH1 | 0.946667 | EVI5 | 0.935 |
| CRADD | 0.960833 | DNAJB6 | 0.946667 | WWP2 | 0.934167 |
| DXO | 0.96 | EIF4B | 0.946667 | KIFBP | 0.934167 |
| DBI | 0.96 | EDF1 | 0.945833 | GCC1 | 0.933333 |
| GP6 | 0.96 | RILP | 0.945833 | CHMP6 | 0.933333 |
| ANXA4 | 0.9575 | CHAC2 | 0.945833 | DTYMK | 0.933333 |
| CA13 | 0.9575 | ARF6 | 0.945833 | PCYT2 | 0.932917 |
| OTUD6B | 0.9575 | CDC26 | 0.945 | PDIA4 | 0.9325 |
| DAG1 | 0.9575 | ANXA11 | 0.945 | YWHAQ | 0.9325 |
| PARK7 | 0.9575 | ASAH1 | 0.945 | PTGES2 | 0.9325 |
| PSMG4 | 0.956667 | NUDT5 | 0.945 | COR01A | 0.9325 |
| NT5C3A | 0.955833 | RAB33A | 0.945 | PIBF1 | 0.931667 |
| IST1 | 0.955833 | AK1 | 0.944167 | PPBP | 0.931667 |
| HTRA2 | 0.955833 | DAB2 | 0.944167 | DCTN6 | 0.931667 |
| CACYBP | 0.955833 | ERP29 | 0.944167 | TSNAX | 0.931667 |
| MCFD2 | 0.955833 | MAPK9 | 0.944167 | LYN | 0.931667 |
| AIFM1 | 0.955833 | SERPINB1 | 0.943333 | GORASP2 | 0.930833 |
| DENR | 0.955 | GTPBP2 | 0.943333 | LAPS | 0.930833 |
| DPY30 | 0.955 | CN PY4 | 0.9425 | TMED8 | 0.930833 |
| MPIG6B | 0.955 | FAM13A | 0.9425 | TNFSF14 | 0.930833 |
| OGA | 0.955 | DA RSI | 0.9425 | TBCC | 0.930833 |
| MDH1 | 0.955 | PRDX1 | 0.9425 | YES1 | 0.93 |
| FKBPL | 0.955 | NUMB | 0.941667 | STIP1 | 0.93 |
| CIAPIN1 | 0.954167 | SYAP1 | 0.941667 | TXNDC9 | 0.93 |
| RGS10 | 0.954167 | STK24 | 0.941667 | TPD52L2 | 0.93 |
| UBAC1 | 0.954167 | JPT2 | 0.941667 | PSMD1 | 0.93 |
| GGCT | 0.954167 | PPP1R2 | 0.941667 | PPP1R14A | 0.93 |
| NUB1 | 0.954167 | NUCB2 | 0.941667 | | |
| FADD | 0.953333 | ATOX1 | 0.941667 | | |
| CRKL | 0.953333 | DRG2 | 0.941667 | | |

### (3) Biomarker candidates for discriminating pancreatic cancer from healthy individual No. 2

Proteins having high specificity for pancreatic cancer in which a two-fold or more expression difference was observed between pancreatic cancer and healthy individuals but no expression difference was observed between colorectal cancer and healthy individuals were defined as "pancreatic cancer-specific biomarkers" (Table 7).

That is, antigens having signal responses worth analyzing were extracted from a list of 3072 antigens in pancreatic cancer and colorectal cancer patient plasma and healthy individual plasma specimens, which were selected without bias such as age and sex from the protein expression list obtained by the PEA method, and proteins were selected for which a two-fold or more expression difference was observed between pancreatic cancer and healthy individuals, the fold change was Log2X>1.0 between colorectal cancer and pancreatic cancer, Log2X<0.65 between colorectal cancer and healthy individuals, and a statistically significant difference of P<0.01 was observed between pancreatic cancer and colorectal cancer, but a significant difference of P<0.01 could not be confirmed between colorectal cancer and healthy individuals.

Proteins having such characteristics were defined as pancreatic cancer biomarkers.

**[Table 7]**

| Pancreatic cancer-specific biomarker | Expression level (Log2X) | | | Expression difference vs Healthy individual | | Pancreatic cancer vs Healthy individual (t-test) | Colorectal cancer vs Healthy individual |
|---|---|---|---|---|---|---|---|
| | Pancreatic cancer | Colorectal cancer | Healthy | Pancreatic cancer - Healthy | Colorectal cancer - Healthy | Pancreatic cancer - Healthy | Colorectal cancer - Healthy |
| GP2 | 3.16773 | 1.170757 | 1.163375 | 2.004355 | 0.007381667 | 6.35913E-06 | 0.980884441 |
| CUZD1 | 2.32101 | 0.744003 | 0.500153 | 1.8208575 | 0.243850833 | 0.000175104 | 0.203292449 |
| CTRL | 2.299283 | 1.090257 | 0.640385 | 1.6588975 | 0.449871667 | 6.26536E-05 | 0.137161827 |
| BST2 | 1.939513 | 0.746947 | 0.64896 | 1.2905525 | 0.097986667 | 0.000760058 | 0.633074724 |
| VAMP5 | 1.710175 | 0.560257 | 0.583843 | 1.1263325 | -0.023585833 | 0.003046411 | 0.802259495 |
| PNLIPRP1 | 0.994775 | -0.09746 | -0.74722 | 1.7419975 | 0.6497625 | 6.75157E-06 | 0.022213748 |
| CPB1 | 1.352758 | 0.311009 | -0.26746 | 1.6202175 | 0.57755 | 3.4512E-06 | 0.010600565 |
| CELA3A | 1.209628 | 0.203973 | 0.089505 | 1.1201225 | 0.114468333 | 0.000408052 | 0.579489117 |
| SULT2A1 | 0.40744 | -0.59451 | -0.61562 | 1.02306 | 0.02111 | 0.008451398 | 0.905623787 |

### (4) Biomarker candidates for discriminating colorectal cancer from healthy individual No. 2

Proteins having high specificity for colorectal cancer in which a four-fold or more expression difference could be confirmed between colorectal cancer and healthy individuals with a statistically significant difference of 1.0×10⁻⁶ or less, but a two-fold or more expression difference was also observed between colorectal cancer and pancreatic cancer were used as colorectal cancer-specific biomarkers (Table 8).

**[Table 8]**

| Colorectal cancer-specific biomarker | Expression level (Log2X) | | | Expression difference vs Healthy individual (Log2X) | | Pancreatic cancer vs Healthy individual (t-test) | Colorectal cancer vs Healthy individual |
|---|---|---|---|---|---|---|---|
| | Pancreatic cancer | Colorectal cancer | Healthy | Pancreatic cancer - Healthy | Colorectal cancer - Healthy | Pancreatic cancer - Healthy | Colorectal cancer - Healthy |
| SMTN | 3.96443 | 5.058117 | 2.731015 | 1.233415 | 2.327101667 | 0.000440928 | 2.62504E-09 |

### (2) Example 2

As a verification of Example 1, a verification experiment was performed using specimens different from those of Example 1. Using 177 cases of plasma specimens of pancreatic cancer, colorectal cancer, chronic pancreatitis, and healthy individuals with matched background information such as sex and age (Table 9), a biomarker candidate list for discriminating healthy individuals was prepared using a proximity extension assay (PEA) method. Table 9 is validation cohort (cohort for validation of predictive variables (comparative study)).

**[Table 9]**

| | Healthy control | Pancreatic cancer | Chronic pancreatitis | Colorectal cancer |
|---|---|---|---|---|
| Sample number | 33 | 74 | 10 | 60 |
| Age (Mean ±SD) | 62.8 ± 8.3 | 62.8 ±11.7 | | 62.8 ± 13.6 |
| Gender (Mail/Female) | 14/19 | 28/46 | | |
| Stage (UICC8th) | | 16 | | 6 |
| I | | | | |
| II | | 8 | | 19 |
| III | | 12 | | 23 |
| IV | | 38 | | 12 |

As a result of narrowing the profiled proteins down to Log2X>Fold change2.5, Student's t-test P<1.0×10⁻⁹, AUC 0.95≤, the top proteins were included in Organ Damage, and thus the verification cohort was analyzed using Olink Target 96 Organ damage panel from Olink.

As a result, it was verified that Training cohort and Validation cohort obtained equivalent results. (Table 10)

**[Table 10]**

| | Colorectal cancer | | Pancreatic cancer | |
|---|---|---|---|---|
| Gene | Training cohort | Validation cohort | Training cohort | Validation cohort |
| BID | 1 | 0.997 | 0.999 | 0.998 |
| FK8P18 | 0.998 | 0.991 | 0.998 | 0.995 |
| AIFM1 | 0.956 | 0.938 | 0.951 | 0.92 |
| VASH1 | 0.956 | 0.956 | 0.951 | 0.922 |
| MAX | 0.948 | 0.951 | 0.939 | 0.927 |
| PVALB | 0.945 | 0.936 | 0.933 | 0.917 |
| BTC | 0.938 | 0.883 | 0.923 | 0.876 |
| NUB1 | 0.938 | 0.934 | 0.922 | 0.932 |
| YES1 | 0.932 | 0.875 | 0.914 | 0.867 |
| TOP2B | 0.928 | 0.945 | 0.91 | 0.914 |
| INPPL1 | 0.919 | 0.836 | 0.896 | 0.836 |
| FOXO1 | 0.919 | 0.868 | 0.894 | 0.867 |
| NUCB2 | 0.917 | 0.94 | 0.892 | 0.928 |
| ITGB1BP1 | 0.913 | 0.876 | 0.886 | 0.893 |
| ERBIN/ER BB2IP | 0.904 | 0.744 | 0.869 | 0.697 |
| BANK1 | 0.888 | 0.813 | 0.84 | 0.842 |
| PRKRA | 0.884 | 0.838 | 0.834 | 0.843 |
| SMAD1 | 0.883 | 0.83 | 0.833 | 0.817 |

### INDUSTRIAL APPLICABILITY

The present invention is useful for diagnosis and the like of pancreatic cancer or colorectal cancer.

### [Sequence Listing]

PCT_Method for assisting diagnosis of pancreatic cancer _20230210_112212_13.xml

## Claims

1. A method for assisting diagnosis of colorectal cancer, comprising:
detecting and/or quantifying at least one biomarker in a body fluid sample derived from a subject,
the biomarker being a protein selected from the group consisting of FKBP1B, BID, EREG, MYDGF, TACC3, TBCA, FKBP14, MIF, ARF6, MESD, CIRBP, MANF, ERP29, PDLIM7, DNM1, STAT5B, GRAP2, UFD1, SNCA, PLCB2, SULT1A1, ABHD14B, PRKAR1A, PPIB, HAGH, GP6, EIF4EBP1, EGF, CRADD, MPIG6B, LAT, CDC37, TWF2, PPP1R12A, CXCL3, MAX, CDKN2D, EIF4E, IST1, CD69, AIFM1, CRKL, CACYBP, CA13, NT5C3A, DAB2, DBNL, CLEC1B, GOPC, TMED8, GCC1, TBCB, ATPSIF1, NFU1, CORO1A, DTD1, CRYZL1, CXCL5, SRC, BCL2L1, PPP2R5A, DIABLO, and RBPMS2, a fragment thereof, or a post-translational modification form thereof.

2. A method for assisting diagnosis of colorectal cancer, comprising:
detecting and/or quantifying at least one biomarker in a body fluid sample derived from a subject,
the biomarker being a protein selected from the group consisting of BID, TXN, FKBP1B, APEX1, ABHD14B, ATXN10, PRKAR1A, EREG, S100A4, AARSD1, TXNDC5, STX3, CDKN2D, MYDGF, ACYP1, PPIB, TBCA, LRPAP1, PEBP1, SEMA4D, MANF, MSRA, TACC3, HAGH, NSFL1C, LACTB2, FKBP14, BNIP2, ADD1, EIF4EBP1, TALDO1, ANXA3, FABP5, TREML1, SNX9, HSPA1A, SLC9A3R1, CXCL3, TWF2, NUDT16, SOD1, PLPBP, CA2, CD69, EGF, PRDX5, RWDD1, BAX, MIF, CRADD, DXO, DBI, GP6, ANXA4, CA13, OTUD6B, DAG1, PARK7, PSMG4, NT5C3A, IST1, HTRA2, CACYBP, MCFD2, AIFM1, DENR, DPY30, MPIG6B, OGA, MDH1, FKBPL, CIAPIN1, RGS10, UBAC1, GGCT, NUB1, FADD, CRKL, NAA10, CLEC1B, PIK3AP1, CXCL6, TXNRD1, LAT, NUDT2, FHIT, CDC37, HPCAL1, DBNL, DNAJB14, FKBP4, TYMP, GLOD4, SIAE, GPI, FIS1, CRYBB1, RABEP1, THTPA, CEP20, SH3GLB2, NMT1, PDCD5, USP25, QDPR, IL7, PDLIM7, RAB2B, PPP1R12A, MAP2K1, TPMT, DFFA, RAB27B, MESD, GMPR2, TBCB, SERPINB6, CIRBP, TMEM106A, TP53I3, CETN3, COMMD1, NRGN, EIF4E, METAP2, KYAT1, DNPH1, DNAJB6, EIF4B, EDF1, RILP, CHAC2, ARF6, CDC26, ANXA11, ASAH1, NUDT5, RAB33A, AK1, DAB2, ERP29, MAPK9, SERPINB1, GTPBP2, CNPY4, FAM13A, DARS1, PRDX1, NUMB, SYAP1, STK24, JPT2, PPP1R2, NUCB2, ATOX1, DRG2, CHMP1A, CCDC134, MAX, STAT5B, MED18, TMSB10, CASP8, GIPC3, SNX5, PACS2, RABGAP1L, DNAJB2, CASP2, HS1BP3, EIF4G1, IPCEF1, SNAP29, SELP, SCARF1, HPSE, AKT3, NAPRT, ASPSCR1, VASH1, PVALB, PSMD9, DTD1, FAM172A, IMPA1, CXCL8, PPIF, ARL2BP, CALCOCO2, TIMM10, INPP1, CASP3, DNM1, AP3B1, GRAP2, HARS1, TXLNA, MAPKAPK2, TOMM20, PDAP1, SRPK2, HHEX, SH3BP1, MTDH, EVI5, WWP2, KIFBP, GCC1, CHMP6, DTYMK, PCYT2, PDIA4, YWHAQ, PTGES2, CORO1A, PIBF1, PPBP, DCTN6, TSNAX, LYN, GORASP2, LAP3, TMED8, TNFSF14, TBCC, YES1, STIP1, TXNDC9, TPD52L2, PSMD1, and PPP1R14A, a fragment thereof, or a post-translational modification form thereof.

3. A method for assisting diagnosis of colorectal cancer, comprising:
detecting and/or quantifying at least one biomarker in a body fluid sample derived from a subject,
the biomarker being a protein that is SMTN, a fragment thereof, or a post-translational modification form thereof.

4. The method of any one of claims 1 to 3, wherein
the body fluid sample is whole blood, serum, plasma, lymph fluid, urine, or saliva.

5. The method of any one of claims 1 to 3, wherein
the biomarker is detected and/or quantified by an immunological measurement method, and
the immunological measurement method is Western blotting, radioimmunoassay (RIA), enzyme immunoassay (ELISA, EIA), luminescence immunoassay, fluorescence immunoassay, or PEA method.

6. A method for assisting diagnosis of pancreatic cancer, comprising:
detecting and/or quantifying at least one biomarker in a body fluid sample derived from a subject,
the biomarker being a protein selected from the group consisting of FKBP1B, BID, EREG, STAT5B, UFD1, SNCA, PLCB2, AKT2, ARF6, MANF, PPP2R5A, MESD, DOK1, GRAP2, TBCA, TACC3, ERP29, ABHD14B, CIRBP, MYDGF, SULT1A1, CRKL, HAGH, PDLIM7, MIF, FKBP14, DNM1, CD69, NT5C3A, SRC, GOPC, CMIP, CA13, TWF2, DTD1, IRAK4, BCL2L1, MPIG6B, CASP3, MPI, LAT, MTSS2, DAB2, PPIB, TMED8, JPT2, PRKAR1A, AIFM1, CACYBP, and TBCB, a fragment thereof, or a post-translational modification form thereof.

7. A method for assisting diagnosis of pancreatic cancer, comprising:
detecting and/or quantifying at least one biomarker in a body fluid sample derived from a subject,
the biomarker being a protein selected from the group consisting of BID, FKBP1B, HAGH, PRKAR1A, ABHD14B, RILP, CD69, ATXN10, APEX1, GOPC, MPIG6B, ARF6, TIMM10, NUDT2, MANF, LAT, TXN, EREG, AARSD1, CASP3, CMIP, PPIB, CDC37, PLPBP, PEBP1, TIMM8A, GP6, EIF4EBP1, JPT2, MTSS2, RGS10, MCFD2, HPCAL1, INPP1, TBCA, DAG1, RWDD1, CDKN2D, NAPRT, MYDGF, ERP29, RAB27B, PCYT2, PRDX5, LACTB2, TALDO1, MSRA, NT5C3A, TACC3, FHIT, CRYGD, CIRBP, TPMT, HSPA1A, DNAJB14, FKBP14, ACYP1, CRYBB1, MESD, MAP2K1, AIFM1, PDCD5, VASH1, SNCA, EGF, SOD1, IMPA1, PARK7, YWHAQ, TYMP, NFKB1, LPP, BNIP2, GET3, MIF, PRDX1, SNX9, ARL13B, HARS1, TWF2, NAA10, BACH1, STX3, FADD, CRADD, PPP1R2, CXCL3, ANXA3, NRGN, PDIA4, FXYD5, AAMDC, CACYBP, PDAP1, ANXA11, LYN, OTUD6B, GLOD4, TMED8, CNPY4, PPP1R14A, VPS37A, CLEC1B, MPI, FAM172A, PLA2G4A, STAT5B, PPME1, SLC9A3R1, MAX, DNAJB6, CASP2, TREML1, CHMP6, CIAPIN1, CASP8, BCL2L1, EIF4B, TRIAP1, DTYMK, DNM1, NUDT5, UFD1, HTRA2, USP25, NSFL1C, DXO, CA13, CRKL, COMMD1, AK1, CHAC2, KIFBP, PLCB2, S100A4, MAPK9, EDF1, PVALB, PDE5A, NUMB, PPP2R5A, IL7, QDPR, TMSB 10, MED 18, and EBAG9, a fragment thereof, or a post-translational modification form thereof.

8. A method for assisting diagnosis of pancreatic cancer, comprising:
detecting and/or quantifying at least one biomarker in a body fluid sample derived from a subject,
the biomarker being a protein selected from the group consisting of GP2, CUZD1, CTRL, BST2, VAMP5, PNLIPRP1, CPB1, CELA3A, and SULT2A1, a fragment thereof, or a post-translational modification form thereof.

9. The method of any one of claims 6 to 8, wherein
the body fluid sample is whole blood, serum, plasma, lymph fluid, urine, or saliva.

10. The method of any one of claims 6 to 8, wherein
the biomarker is detected and/or quantified by an immunological measurement method, and
the immunological measurement method is Western blotting, radioimmunoassay (RIA), enzyme immunoassay (ELISA, EIA), luminescence immunoassay, fluorescence immunoassay, or PEA method.

11. A biomarker for detecting pancreatic cancer, comprising:
at least one protein selected from the group consisting of FKBP1B, BID, EREG, STAT5B, UFD1, SNCA, PLCB2, AKT2, ARF6, MANF, PPP2R5A, MESD, DOK1, GRAP2, TBCA, TACC3, ERP29, ABHD14B, CIRBP, MYDGF, SULT1A1, CRKL, HAGH, PDLIM7, MIF, FKBP14, DNM1, CD69, NT5C3A, SRC, GOPC, CMIP, CA13, TWF2, DTD1, IRAK4, BCL2L1, MPIG6B, CASP3, MPI, LAT, MTSS2, DAB2, PPIB, TMED8, JPT2, PRKAR1A, AIFM1, CACYBP, and TBCB, a fragment thereof, or a post-translational modification form thereof.

12. A biomarker for detecting pancreatic cancer, comprising:
at least one protein selected from the group consisting of BID, FKBP1B, HAGH, PRKAR1A, ABHD14B, RILP, CD69, ATXN10, APEX1, GOPC, MPIG6B, ARF6, TIMM10, NUDT2, MANF, LAT, TXN, EREG, AARSD1, CASP3, CMIP, PPIB, CDC37, PLPBP, PEBP1, TIMM8A, GP6, EIF4EBP1, JPT2, MTSS2, RGS10, MCFD2, HPCAL1, INPP1, TBCA, DAG1, RWDD1, CDKN2D, NAPRT, MYDGF, ERP29, RAB27B, PCYT2, PRDX5, LACTB2, TALDO1, MSRA, NT5C3A, TACC3, FHIT, CRYGD, CIRBP, TPMT, HSPA1A, DNAJB14, FKBP14, ACYP1, CRYBB1, MESD, MAP2K1, AIFM1, PDCD5, VASH1, SNCA, EGF, SOD1, IMPA1, PARK7, YWHAQ, TYMP, NFKB1, LPP, BNIP2, GET3, MIF, PRDX1, SNX9, ARL13B, HARS1, TWF2, NAA10, BACH1, STX3, FADD, CRADD, PPP1R2, CXCL3, ANXA3, NRGN, PDIA4, FXYD5, AAMDC, CACYBP, PDAP1, ANXA11, LYN, OTUD6B, GLOD4, TMED8, CNPY4, PPP1R14A, VPS37A, CLEC1B, MPI, FAM172A, PLA2G4A, STAT5B, PPME1, SLC9A3R1, MAX, DNAJB6, CASP2, TREML1, CHMP6, CIAPIN1, CASP8, BCL2L1, EIF4B, TRIAP1, DTYMK, DNM1, NUDT5, UFD1, HTRA2, USP25, NSFL1C, DXO, CA13, CRKL, COMMD1, AK1, CHAC2, KIFBP, PLCB2, S100A4, MAPK9, EDF1, PVALB, PDE5A, NUMB, PPP2R5A, IL7, QDPR, TMSB 10, MED 18, and EBAG9, a fragment thereof, or a post-translational modification form thereof.

13. A biomarker for detecting pancreatic cancer, comprising:
at least one protein selected from the group consisting of GP2, CUZD1, CTRL, BST2, VAMP5, PNLIPRP1, CPB1, CELA3A, and SULT2A1, a fragment thereof, or a post-translational modification form thereof.

14. A biomarker for detecting colorectal cancer, comprising:
at least one protein selected from the group consisting of BID, FKBP1B, EREG, MYDGF, TACC3, TBCA, FKBP14, MIF, ARF6, MESD, CIRBP, MANF, ERP29, PDLIM7, DNM1, STAT5B, GRAP2, UFD1, SNCA, PLCB2, SULT1A1, ABHD14B, PRKAR1A, PPIB, HAGH, GP6, EIF4EBP1, EGF, CRADD, MPIG6B, LAT, CDC37, TWF2, PPP1R12A, CXCL3, MAX, CDKN2D, EIF4E, IST1, CD69, AIFM1, CRKL, CACYBP, CA13, NT5C3A, DAB2, DBNL, CLEC1B, GOPC, BCL2L1, PPP2R5A, DIABLO, and RBPMS2, a fragment thereof, or a post-translational modification form thereof.

15. A biomarker for detecting colorectal cancer, comprising:
at least one protein selected from the group consisting of BID, TXN, FKBP 1B, APEX1, ABHD14B, ATXN10, PRKAR1A, EREG, S100A4, AARSD1, TXNDC5, STX3, CDKN2D, MYDGF, ACYP1, PPIB, TBCA, LRPAP1, PEBP1, SEMA4D, MANF, MSRA, TACC3, HAGH, NSFL1C, LACTB2, FKBP14, BNIP2, ADD1, EIF4EBP1, TALDO1, ANXA3, FABP5, TREML1, SNX9, HSPA1A, SLC9A3R1, CXCL3, TWF2, NUDT16, SOD1, PLPBP, CA2, CD69, EGF, PRDX5, RWDD1, BAX, MIF, CRADD, DXO, DBI, GP6, ANXA4, CA13, OTUD6B, DAG1, PARK7, PSMG4, NT5C3A, IST1, HTRA2, CACYBP, MCFD2, AIFM1, DENR, DPY30, MPIG6B, OGA, MDH1, FKBPL, CIAPIN1, RGS10, UBAC1, GGCT, NUB1, FADD, CRKL, NAA10, CLEC1B, PIK3AP1, CXCL6, TXNRD1, LAT, NUDT2, FHIT, CDC37, HPCAL1, DBNL, DNAJB14, FKBP4, TYMP, GLOD4, SIAE, GPI, FIS1, CRYBB1, RABEP1, THTPA, CEP20, SH3GLB2, NMT1, PDCD5, USP25, QDPR, IL7, PDLIM7, RAB2B, PPP1R12A, MAP2K1, TPMT, DFFA, RAB27B, MESD, GMPR2, TBCB, SERPINB6, CIRBP, TMEM106A, TP53I3, CETN3, COMMD1, NRGN, EIF4E, METAP2, KYAT1, DNPH1, DNAJB6, EIF4B, EDF1, RILP, CHAC2, ARF6, CDC26, ANXA11, ASAH1, NUDT5, RAB33A, AK1, DAB2, ERP29, MAPK9, SERPINB1, GTPBP2, CNPY4, FAM13A, DARS1, PRDX1, NUMB, SYAP1, STK24, JPT2, PPP1R2, NUCB2, ATOX1, DRG2, CHMP1A, CCDC134, MAX, STAT5B, MED18, TMSB10, CASP8, GIPC3, SNX5, PACS2, RABGAP1L, DNAJB2, CASP2, HS1BP3, EIF4G1, IPCEF1, SNAP29, SELP, SCARF1, HPSE, AKT3, NAPRT, ASPSCR1, VASH1, PVALB, PSMD9, DTD1, FAM172A, IMPA1, CXCL8, PPIF, ARL2BP, CALCOCO2, TIMM10, INPP1, CASP3, DNM1, AP3B1, GRAP2, HARS1, TXLNA, MAPKAPK2, TOMM20, PDAP1, SRPK2, HHEX, SH3BP1, MTDH, EVI5, WWP2, KIFBP, GCC1, CHMP6, DTYMK, PCYT2, PDIA4, YWHAQ, PTGES2, CORO1A, PIBF1, PPBP, DCTN6, TSNAX, LYN, GORASP2, LAP3, TMED8, TNFSF14, TBCC, YES1, STIP1, TXNDC9, TPD52L2, PSMD1, and PPP1R14A, a fragment thereof, or a post-translational modification form thereof.

16. A biomarker for detecting colorectal cancer, comprising:
SMTN or a fragment thereof, or a post-translational modification form thereof.
